# EUROPÄISCHE PATENTSCHRIFT

(11) **EP 1 917 290 B1**
(45) Veröffentlichungstag und Bekanntmachung des Hinweises auf die Patenterteilung: **26.06.2013**
(21) Anmeldenummer: 06778073.4
(22) Anmeldetag: 31.07.2006
(51) Int. Cl.: C08G 77/04, C08G 77/26, D06M 15/643, A61K 8/89, A61K 8/898

(54) **POLYAMMONIUM-POLYSILOXANCOPOLYMERE**
POLYAMMONIUM-POLYSILOXANE COPOLYMERS
COPOLYMERES DE POLYAMMONIUM-POLYSILOXANE

(30) Priorität: 01.08.2005 DE 102005036602
(43) Veröffentlichungstag der Anmeldung: 07.05.2008
(73) Patentinhaber: Momentive Performance Materials GmbH, 51368 Leverkusen (DE)
(72) Erfinder: WAGNER, Roland, 53721 Siegburg (DE); LANGE, Horst, 44879 Bochum (DE); WITOSSEK, Anita, 40764 Langenfeld (DE); STACHULLA, Karl-Heinz, 51379 Leverkusen (DE); SOCKEL, Karl-Heinz, 51373 Leverkusen (DE); KROPFGANS, Martin, 51519 Odenthal (DE)
(74) Vertreter: Roos, Peter
(86) Internationale Anmeldenummer: PCT/EP2006/064833
(87) Internationale Veröffentlichungsnummer: WO 2007/014930

(56) Entgegenhaltungen:
- WO-A-02/10259
- DE-A1- 10 214 290
- US-A1- 2004 048 996

## Beschreibung

Die Erfindung betrifft Polyammonium-Polysiloxancopolymere, Verfahren zu ihrer Herstellung sowie ihre Verwendung.

Aus der WO 2004/042136 sind lineare Polyamino- und/oder Polyammonium-Polysiloxancopolymere bekannt. Die dort beschriebenen Copolymere sind insbesondere hinsichtlich ihrer Substantivität, d.h. der Fähigkeit, möglichst lange, insbesondere in Gegenwart von stark tensidhaltigen Zusammensetzungen an der Oberfläche verschiedenster Substrate zu haften, um dort die gewünschten Effekte, insbesondere ihre weichmachenden und gegebenenfalls hydrophilierenden Effekte, auszuüben, noch verbesserungswürdig.

Die WO 0210259 offenbart Polysiloxanpolymere vorzugsweise zur Verwendung als waschbeständige hydrophile Weichmacher auf Basis quartärer Ammoniumgruppen enthaltender Siloxane, Verfahren zu deren Herstellung und deren Verwendung vorzugsweise als Weichmacher. Die Polysiloxanpolymere weisen in der Polymerhauptkette quaternäre Ammoniumgruppen auf, die jedoch keine weiteren tertiäre Amingruppen, quaternäre Ammonium- bzw. Aminoxidgruppen tragen.

Die Erfinder stellten sich somit die Aufgabe, neue Polyammonium-Polysiloxancopolymere bereitzustellen, die über neuartige Eigenschaftsprofile verfügen. Überraschend fanden die Erfinder, dass neue insbesondere lineare Polyammonium-Polysiloxancopolymere aus deren Polymerhauptkette in hoher Dichte quaternäre Ammoniumgruppen aufweisende Seitenketten herausragen, über neuartige Eigenschaften verfügen.

Die vorliegende Erfindung stellt somit Polyammonium-Polysiloxancopolymere, enthaltend Wiederholungseinheiten der Formel (I) bereit: 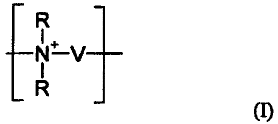 worin R jeweils unabhängig voneinander organische Gruppen sind, die jeweils mindestens eine Gruppe enthalten, die aus quaternären Ammoniumgruppen oder Aminoxidgruppen ausgewählt sind,
V ausgewählt wird aus der Gruppe V¹ und der Gruppe V²,
V² ausgewählt wird aus zweiwertigen oder dreiwertigen, geradkettigen, cyclischen oder verzweigten, gesättigten, ungesättigten oder aromatischen Kohlenwasserstoffresten mit bis zu 1000 Kohlenstoffatomen (wobei die Kohlenstoffatome des unten definierten Polysiloxanrestes Z² nicht mitgezählt werden), die gegebenenfalls eine oder mehrere Gruppen, ausgewählt aus
-O-,
-NR²-,
-N⁺R²₂-,
worin R² Wasserstoff, einen einwertigen, geradkettigen, cyclischen oder verzweigten, gesättigten, ungesättigten oder aromatischen Kohlenwasserstoffrest mit bis zu 100 Kohlenstoffatomen darstellt, der eine oder mehrere Gruppen ausgewählt aus -O-, -NH-, -C(O)- und - C(S)- enthalten kann, und der gegebenenfalls durch eine oder mehrere Substituenten, ausgewählt aus der Gruppe, die besteht aus einer Hydroxylgruppe, einer gegebenenfalls substituierten, bevorzugt ein oder mehrere Stickstoffatome enthaltenden heterocyclischen Gruppe, Amino, Alkylamino, Dialkylamino, Polyetherresten und Polyetheresterresten substituiert sein kann, wobei wenn mehrere Gruppen -NR²- vorliegen, diese gleich oder verschieden sein können,
-C(O)-,
-C(S)-
enthalten kann, und
der Rest V² gegebenenfalls durch eine oder mehrere Hydroxylgruppen substituiert sein kann, und
der Rest V² mindestens eine Gruppe -Z²- der Formel 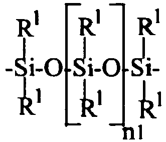 enthält, worin
R¹ gleich oder verschieden sein kann und aus der Gruppe ausgewählt wird, die besteht aus: C₁ bis C₂₂-Alkyl, Fluor(C₁-C₁₀)-alkyl und C₆-C₁₀-Aryl, und
n₁ = 20 bis 1000 bedeutet,
V¹ ausgewählt wird aus zweiwertigen oder dreiwertigen, geradkettigen, cyclischen oder verzweigten, gesättigten, ungesättigten oder aromatischen Kohlenwasserstoffresten mit bis zu 1000 Kohlenstoffatomen, die gegebenenfalls eine oder mehrere Gruppen, ausgewählt aus
-O-,
-NR²-,
-N⁺R²₂-,
worin R² wie oben definiert ist, und wobei die Gruppen R² in den Gruppen V¹ und V² gleich oder verschieden sein können,
-C(O)-,
-C(S)- und
-Z¹- enthalten kann, worin -Z¹- eine Gruppe der Formel 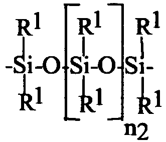 ist, worin
R¹ wie oben definiert ist, wobei die Gruppen R¹ in den Gruppen V¹ und V² gleich oder verschieden sein können, und n₂ = 0 bis 19 bedeutet,
und der Rest V¹ gegebenenfalls durch eine oder mehrere Hydroxylgruppen substituiert sein kann,
worin die Gruppen V¹ und V² in den Polyammonium-Polysiloxancopolymeren gleich oder verschieden sein können, mit der Maßgabe, dass wenigstens eine Gruppe Z¹ oder Z² vorhanden ist, und
worin die aus den Ammoniumgruppen resultierenden positiven Ladungen durch organische oder anorganische Säureanionen neutralisiert sind. Die Reste R stellen in einer bevorzugten Ausführungsform Betainreste dar.

Die erfindungsgemäßen Polyammonium-Polysiloxancopolymere umfassen zweckmäßig mindestens zwei bevorzugt mehr als zwei Wiederholungseinheiten der Formel

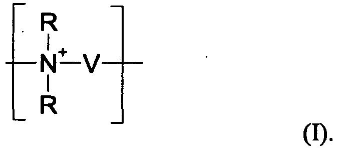

Bei zwei Wiederholungseinheiten ergibt sich also folgende Struktur: 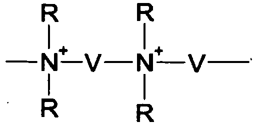 und bei drei Wiederholungseinheiten folgende Struktur: 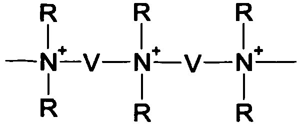 usw. Die Absättigung der terminalen Stickstoffatome erfolgt dabei durch beliebige einwertige organische Reste, wobei insbesondere die für R² genannten Reste erwähnt werden können. Die Art der terminalen Gruppen ergibt sich im allgemeinen aus der Art der verwendeten Ausgangsmaterialien bzw. Monomere oder gegebenenfalls aus einwertigen sogenannten Stoppermolekülen, die der Reaktionsmischung zugesetzt werden können.

Organische oder anorganische Säureanionen sind beispielsweise deprotonierte organische und anorganische Säuren, wie deprotonierte Carbonsäuren, d.h. Carboxylatanionen, wie Acetat, Propionat, Octanoat, Decanoat, Dodecanoat, Tetradecanoat, Hexadecanoat, Octadecanoat, Oleat, Citrat und Benzoat, Polyethercarboxylate, Polyethersulfate, Alkylsulfate, Alkylsulfonate und Arylsulfonate, oder deprotonierte Mineralsäuren, wie Halogenide, insbesondere Chlorid, Sulfate, Phosphate, Nitrate, Bromid, Hydrogensulfat,

In einer bevorzugten Ausführungsform der erfindungsgemäßen Polyammonium-Polysiloxancopolymere werden die quaternären Ammoniumgruppen aufweisenden Reste R aus Gruppen der Formel (II) ausgewählt: 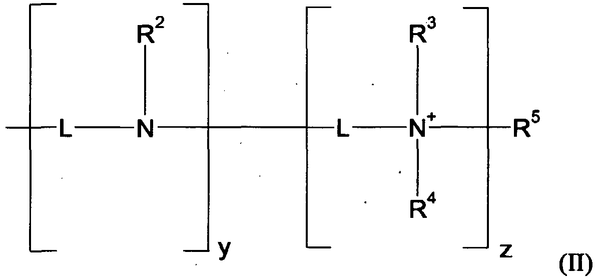 worin
L jeweils unabhängig voneinander ein zweiwertiger geradkettiger, cyclischer oder verzweigter, gesättigter, ungesättigter oder aromatischer Kohlenwasserstoffrest mit bis zu 30 Kohlenstoffatomen darstellt, der eine oder mehrere Gruppen ausgewählt aus -O-, -NH-, -C(O)- und -C(S)- enthalten kann, und der gegebenenfalls durch eine oder mehrere Substituenten, ausgewählt aus der Gruppe, die besteht aus einer Hydroxylgruppe, einer Carboxylgruppe, einer Carboxylatgruppe, einer gegebenenfalls substituierten, bevorzugt ein oder mehrere Stickstoffatome enthaltenden heterocyclischen Gruppe, Amino, Alkylamino, Dialkylamino, Ammoniumgruppe, Polyetherresten und Polyetheresterresten substituiert sein kann,

R² wie oben definiert ist, bevorzugt ausgewählt wird: aus einwertigen, geradkettigen, cyclischen oder verzweigten, gesättigten, ungesättigten oder aromatischen Kohlenwasserstoffresten mit bis zu 30 Kohlenstoffatomen, die eine oder mehrere Gruppen ausgewählt aus -O-, -NH-, -C(O)- und -C(S)- enthalten können, und gegebenenfalls durch einen oder mehrere Substituenten, ausgewählt aus der Gruppe, die besteht aus einer Hydroxylgruppe, einer Carboxylgruppe, einer Carboxylatgruppe, einer gegebenenfalls substituierten, bevorzugt ein oder mehrere Stickstoffatome enthaltenden heterocyclischen Gruppe, Amino, Alkylamino, Dialkylamino, Ammoniumgruppe, Polyetherresten und Polyetheresterresten substituiert sein können,
R³, R⁴ und R⁵ jeweils unabhängig voneinander ausgewählt sind aus einwertigen, geradkettigen, cyclischen oder verzweigten, gesättigten, ungesättigten oder aromatischen Kohlenwasserstoffresten mit bis zu 30 Kohlenstoffatomen, die eine oder mehrere Gruppen ausgewählt aus -O-, -NH-, -C(O)- und -C(S)- enthalten können, und gegebenenfalls durch einen oder mehrere Substituenten, ausgewählt aus der Gruppe, die besteht aus einer Hydroxylgruppe, einer Carboxylgruppe, einer Carboxylatgruppe, einer gegebenenfalls substituierten, bevorzugt ein oder mehrere Stickstoffatome enthaltenden heterocyclischen Gruppe, Amino, Alkylamino, Dialkylamino, Ammoniumgruppe, Polyetherresten und Polyetheresterresten substituiert sein können, und
R³ oder R⁴ Sauerstoff sein können unter Bildung eines Aminoxids, und
z von 1 bis 10 ist und y von 0 bis 10 sein kann.

In dem Rest der allgemeinen Formel (II) können die mit den Indizes y und z versehenen Reste grundsätzlich in beliebiger Anordung zueinander stehen, wie beispielsweise statistisch oder blockartig. In jedem Fall jedoch weist der Rest der allgemeinen Formel (II) mindestens eine quaternäre Ammoniumgruppe oder eine Aminoxidgruppe auf (z ≧ 1), wobei die quaternäre Ammoniumgruppe bevorzugt eine terminale Gruppe der allgemeinen im Folgenden beschriebenen Formel (III) darstellt.

In einer weiteren bevorzugten Ausführungsform der erfindungsgemäßen Polyammonium-Polysiloxancopolymere ist der Rest R jeweils eine Gruppe der Formel (III): 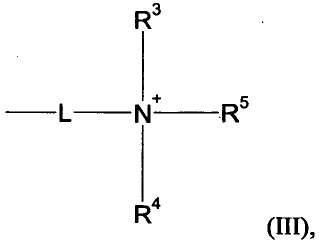 worin L, R³, R⁴ und R⁵ wie oben definiert sind.

In einer weiteren bevorzugten Ausführungsform der erfindungsgemäßen Polyammonium-Polysiloxancopolymere ist der Rest R jeweils mindestens eine Aminoxid-Gruppe der Formel (IV): 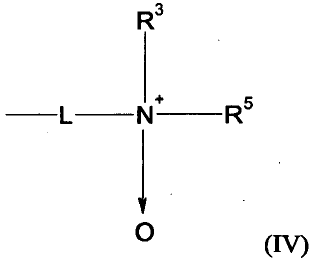 worin L, R³ und R⁵ wie oben definiert sind.

Bevorzugt wird L ausgewählt aus C₁ bis C₁₀-Alkylengruppen, die linear, verzweigt, oder cyclisch sein können, wie beispielsweise Methylen, Ethylen, n-Propylen, n-Butylen etc.

In einer bevorzugten Ausführungsform der Erfindung weisen die Polyammonium-Polysiloxancopolymere mindestens zwei Wiederholungseinheiten V, besonders bevorzugt mindestens wenigstens zwei Wiederholungseinheiten V² auf.

In einer bevorzugten Ausführungsform der Erfindung ist V² eine Gruppe der folgenden Formel

-V²*-Z²-V²*-

ist, worin Z² wie oben definiert ist und V²* einen zweiwertigen geradkettigen, cyclischen oder verzweigten, gesättigten, ungesättigten oder aromatischen Kohlenwasserstoffrest mit bis zu 40 Kohlenstoffatomen darstellt, der gegebenenfalls eine oder mehrere Gruppen, ausgewählt aus -O-, -NR²-, -N⁺R²₂-, worin R² wie oben definiert ist, -C(O)- und -C(S)- enthalten kann, und der Rest V²* gegebenenfalls durch eine oder mehrere Hydroxylgruppen substituiert sein kann.

In diesem Zusammenhang wird klarstellend darauf hingewiesen, dass im Rahmen der vorliegenden Erfindung die Gruppen V, V¹, V² oder V^{2*} ebenfalls quaternäre Ammoniumgruppen -N⁺R²₂- enthalten können. Der Rest R² enthält jedoch keine quaternäre Ammoniumgruppen. Dadurch ist klar unterschieden zwischen dem Rest 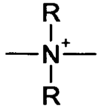 wie er in Formel (I) enthalten ist und dem Rest -N⁺R²₂-, wie er in V enthalten sein kann. Ein in V enhaltender Rest -N⁺R²₂- kann insbesondere eine Dimethylammoniumgruppe sein.

Die Gruppe V¹ wird bevorzugt ausgewählt aus zweiwertigen, geradkettigen, cyclischen oder verzweigten, gesättigten, ungesättigten oder aromatischen Kohlenwasserstoffresten mit bis zu 600 Kohlenstoffatomen, die gegebenenfalls eine oder mehrere Gruppen, ausgewählt aus -O-, -NR²-, -N⁺R²₂-, worin R² wie oben definiert ist, - C(O)-, -C(S)- und -Z¹- enthalten können, worin -Z¹- eine Gruppe der Formel 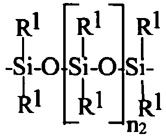 ist, worin
R¹ C₁ bis C₃-Alkyl, Fluor(C₃-C₆)-alkyl oder C₆-Aryl ist, und
n₂ wie oben definiert ist.

Die vorliegende Erfindung betrifft weiterhin Verbindungen der Formel (V): 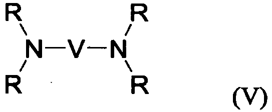 worin R und V wie oben definiert sind, mit der Maßgabe, dass V Z¹ oder Z² enthält, oder ein Säureadditionssalz davon. Säureadditionssalze meint, das mindestens eines der beiden in der allgemeinen Formel (V) sichtbaren Stickstoffatome durch Zusatz einer geeigneten Säure unter Ausbildung des genannten Säureadditionssalz protoniert wird. Geeignete Säuren sind insbesondere Carbonsäuren, wie Essigsäure, und anorganische Mineralsäuren, wie Salzsäure, Schwefelsäure, Phosphorsäure, Salpetersäure etc.

Die Verbindungen der Formel (V) stellen neue Zwischenprodukte der erfindungsgemäßen Polyammonium-Polysiloxancopolymere dar. Sie weisen demnach in den organischen Resten R quaternäre Ammoniumgruppen oder Aminoxidgruppen auf und können mittels bifunktioneller Quaternierungsmittel, wie weiter unten beschrieben, in die erfindungsgemäßen Polyammonium-Polysiloxancopolymere überführt werden. Darüber hinaus wurde erfindungsgemäß festgestellt, dass diese Verbindungen selbst, offenbar aufgrund ihrer hohen Dichte an quaternären Ammoniumgruppen oder Aminoxidgruppen, ebenfalls für die erfindungsgemäßen Anwendungen verwendet werden können. Die Herstellung der Verbindungen der Formel (V) oder ihrer Säureadditionssalze gelingt beispielsweise durch Umsetzung eines Polydimethylsiloxans mit zwei terminalen Epoxygruppen und einem Triamin mit einer innenständiger sekundären Aminogruppe und zwei aussenständigen tertiären Aminogruppen unter Bildung der Verbindung der Formel (V') und anschließender Quaternierung der resultierenden vier tertiären Aminogruppen, wie im Folgenden schematisch gezeigt:

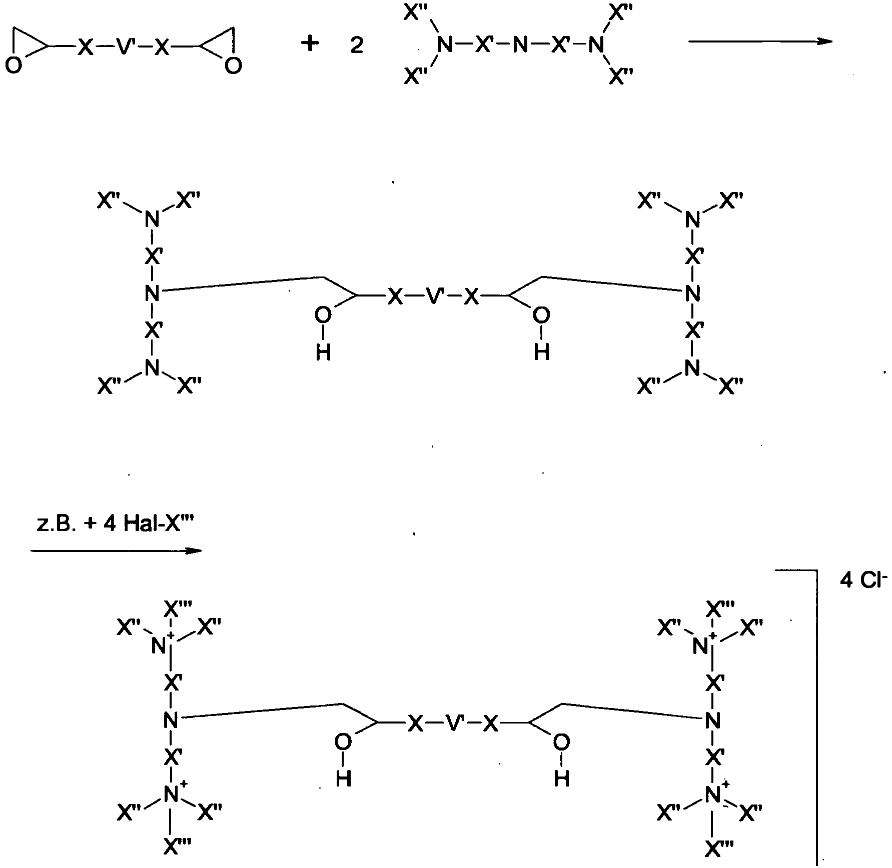

Darin sind X und X' zweiwertige organische Reste, V' ist ein zweiwertiger organischer Rest, der mindestens eine Gruppe Z¹ oder Z² aufweist, und X" und X"' sind einwertige organische Reste.

Die Erfindung betrifft weiterhin die Verbindungen der Formel (V'): 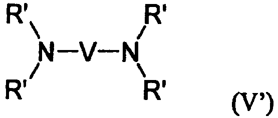 worin R' jeweils eine organische Gruppe ist, die jeweils mindestens eine tertiäre Aminogruppe enthält, und V wie oben defniert ist, oder ein Säureadditionssalz davon. Diese Verbindungen stellen ebenfalls Zwischenprodukte bei der Herstellung der erfindungsgemäßen Polyammonium-Polysiloxancopolymere dar, wobei insbesondere ihre Säureadditionssalze auch in den erfindungsgemäßen Anwendungen verwendet werden können.

In den Verbindungen der Formel (V) und (V') ist V bevorzugt V², weist also bevorzugt den langkettigen Polysiloxanrest Z² auf.

Die Erfindung betrifft weiterhin ein Verfahren zur Herstellung der Polyammonium-Polysiloxancopolymere, worin die Verbindung der Formel (V) mit mindestens einem bifunktionellen (gegebenenfalls auch höher funktionellen) Quaternisierungsmittel umgesetzt wird. Das bifunktionelle Quaternisierungsmittel wird bevorzugt ausgewählt aus Verbindungen, die zwei funktionelle Gruppen aufweisen, die aus der Gruppe ausgewählt werden, die aus Epoxygruppen und Bishalogenalkylgruppen besteht

Ausgangspunkt für die Synthesen der erfindungsgemäßen Substanzen sind insbesondere alpha,omega-SiH funktionalisierte Siloxane der allgemeinen Struktur 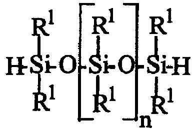 wobei R¹ die oben angegebene Bedeutungen besitzt und n n1 oder n2 sein kann. Sofern nicht kommerziell erhältlich, können diese Siloxane nach bekannten Verfahren, z.B. durch Äquilibrierung hergestellt werden (Silicone, Chemie und Technologie, Vulkan-Verlag, Essen 1989, S. 82-84).

Geeignete Ausgangsstoffe zur Erzeugung von bifunktionellen Alkylierungsmittel sind beispielsweise halogenierte Alkene, speziell Allylchlorid und Allylbromid, ungesättigte Halogencarbonsäureester, speziell Chloressigsäureallylester, Chloressigsäurepropargylester, 3-Chlorpropionsäureallylester und 3-Chlorpropionsäurepropargylester und epoxyfunktionelle Alkene, beispielsweise Vinylcyclohexenoxid und Allylglycidether. Die allgemeine Durchführung von Hydrosilylierungen mit Vertretern der genannten Stoffgruppen ist ebenfalls bekannt (B.Marciniec, Comprehensive Handbook on Hydrosilylation, Pergamon Press, Oxford 1992, S. 116-121, 127-130, 134-137, 151-155).

In einem ersten Schritt können die bifunktioneller Alkylierungsmittel dann mit insbesondere sekundär-tertiären Polyaminen zur Reaktion gebracht werden, wobei die resultierenden Produkte an dem sekundären N-Atom alkyliert werden, wodurch tertiäre N-Atome resultieren. Geeignete Vertreter von sekundär-tertiären Polyaminen sind beispielsweise N,N,N',N'-Tetramethyldiethylentriamin, N,N,N',N'-Tetramethyl-propylentriamin, N,N,N',N'-Tetramethyltriethylentetramin, N,N,N',N'-Tetramethyl-tripropylentetramin, N,N,N',N'-Tetramethyltetraethylenpentamin und N,N,N',N'-Tetramethyltetrapropylenpentamin. Anschließend erfolgt im allgemeinen die Umsetzung mit monofunktionellen Alkylierungsmitteln, derart, dass mindestens zwei tertiäre Aminogruppen im Molekül verbleiben, die anschließend mit einem bifunktionellen Alkylierungs- bzw. Quaternierungsmittel zu den erfindungsgemäßen Polyammonium-Polysiloxan-Verbindungen umgesetzt werden können.

Alternativ können die Alkylierungsschritte auch in anderer Reihenfolge bzw. durch gleichzeitige Zugabe von Mono- und bifunktionellen Alkylierungsmitteln mit geeigneter Stöchiometrie erfolgen.

Als monofunktionelle Quaternierungsagenzien für die alpha, omega-polytertiäraminomodifizierten Polysiloxane kommen die bekannten Alkylierungsagenzien, wie Alkylhalogenide, ganz speziell C₁ bis C₁₂-Alkylhalogenide, oder Dialkylsulfate, ganz speziell Dimethylsulfat, oder Epoxide in Gegenwart von stöchiometrischen Mengen an Säuren HX, speziell Etylenoxid, Propylenoxid, Hexenoxid, Allylglycidether, Isopropylglycidether und Methacrylsäureglycidester oder Halogencarbonsäureester in Betracht.

Die Halogencarbonsäureester werden bevorzugt aus den entsprechenden Alkoholen bzw. niedermolekularen, oligomeren und polymeren Alkylenoxiden der allgemeinen Zusammensetzung

HO[CH₂CH₂O]_{q}-[CH₂CH(CH₃)O]ᵣR⁷

wobei q, r und R⁷ die oben angegebenen Bedeutungen aufweisen, synthetisiert. Bevorzugte alkoxylierte Vertreter sind die entsprechend monosubstituierten Derivate von Diethylenglycol, Triethylenglycol, Tetraethylenglycol, der Oligoethylenglycole mit Molgewichten von 300 bis 1000 g/mol, speziell 400, 600 und 800, sowie Dipropylenglycol. Die Herstellung dieser Ether und Ester erfolgt in bekannter Weise durch sauer oder alkalisch katalysierte Addition von Ethylenoxid und/oder Propylenoxid an die entsprechenden Alkohole (US 5 625 024) oder Carbonsäuren (E.Sung, W. Umbach, H. Baumann, Fette Seifen Anstrichmittel 73, 88 [1971]).

Die nachfolgende Synthese der Halogencarbonsäureester erfolgt in an sich bekannter Weise durch Reaktion mit den C₂ bis C₄-Halogencarbonsäuren, deren Anhydriden oder Säurechloriden.

Die selektive Synthese hydroxyfunktioneller Halogencarbonsäureester (R⁴ = H) gelingt durch Addition von Ethylenoxid und/oder Propylenoxid an die entsprechenden Halogencarbonsäuren unter sauren Bedingungen.

Die in alpha,omega-Stellung polytertiäraminofunktionalisierten Siloxanderivate und die monofunktionellen Quaternierungsagenzien werden in einem molaren Verhältnis umgesetzt, welches dem angestrebten Quaternierungsmuster entspricht, wobei das molare Verhältnis ∑ tertiäres N : Alkylierungsagenz ≥ 1 : (1 - 1 mol tertiäres N), bevorzugt Σ tertiäres N : Alkylierungsagenz = 1 : (1 - 1 mol tertiäres N) beträgt. Bei Vorhandensein von z.B. drei tertiären Aminofunktionen am Siloxankettenende werden folglich maximal zwei dieser tertiären Aminogruppen mit monofunktionellen Agenzien alkyliert.

Die Menge an Alkylierungsagenz muß erhöht werden, wenn noch vorhandene sekundäre Aminofunktionen zusätzlich alkyliert werden sollen. Dies kann beispielsweise bei Verwendung der entsprechenden Trialkylentetramine oder Tetraalkylenpentamine der Fall sein, da für deren einleitende Addition in alpha, omega-Stellung an das reaktive Siloxan nur jeweils eine sekundäre Aminogruppe benötigt wird. Durch Verwendung solcher höheren Polyamine kann die Dichte an quaternierten Gruppen weiter gesteigert werden. Auch in diesen Fällen muß mindestens eine tertiäre Aminogruppe pro Kettenende für die abschließende Copolymersynthese zur Verfügung bleiben.

Geeignete difunktionelle Alkylierungsagenzien sind beispielsweise Dihalogenalkane, wie 1,3-Dichchlorpropan, 1,4-Dichlorbutan und 1,6-Dichlorhexan, Dihalogenalkene und Dihalogenalkine, z.B. 1,4-Dichlorbut(2)in alpha, omega-halogensubstituierte Oligoalkylenoxide, die Diester von Halogencarbonsäuren mit Alkandiolen, Alkendiolen oder Alkindiolen, wie z.B. 1,4-But(2-in)ol, Oligoalkylenoxiden oder zwei primäre OH-Gruppen aufweisenden Polyolen, z.B. Sorbitol, sowie die Diepoxyderivate von Alkanen oder die Diepoxyetherderivate, speziell Glycidetherderivate, von Alkandiolen und Oligoalkylenoxiden,. Die Quaternierungen mit Epoxiden erfolgen in Gegenwart von äquivalenten Mengen Säuren HX.

Die in einer Ausführungsform bevorzugte Einführung von hydrophilierenden Alkylenoxidblöcken gelingt bevorzugt über die entsprechenden Halogencarbonsäureester oder Glycidether der Alkylenoxide. Bevorzugte Ausgangsmaterialien für deren Synthese sind niedermolekulare, oligomere und polymere Alkylenoxide der allgemeinen Zusammensetzung

HO[CH₂CH₂O]_{b}-[CH₂CH(CH₃)O]_{c}H

wobei b und c die oben angegebenen Bedeutungen aufweisen. Bevorzugte Vertreter hinsichtlich des Alkylenoxidblockes sind Diethylenglycol, Triethylenglycol, Tetraethylenglycol, die Oligoethylenglycole mit Molgewichten von 300 bis 1000 g/mol, speziell 400, 600 und 800, sowie Dipropylenglycol.
alpha, omega-Glycidether sind durch Reaktion von Epichlorhydrin mit den entsprechenden Alkylenoxiden in Gegenwart von Alkalihydroxiden zugänglich.

Die Veresterung der Alkylenoxide erfolgt in an sich bekannter Weise durch Reaktion mit den C₂- bis C₄-Halogencarbonsäuren, deren Anhydriden oder Säurechloriden.

Bevorzugt werden die Säurechloride der Chloressigsäure und 3-Chlorpropionsäure eingesetzt und die Reaktion in Abwesenheit von Lösungsmitteln durchgeführt.

In einer speziellen Ausführungsform dienen die vorstehend bereits näher erläuterten reaktiven difunktionellen Zwischenstufen, wie alpha, omega-halogenalkylsubstituierte Siloxane, alpha, omega-halogencarbonsäureestersubstituierte Siloxanen oder alpha, omega-epoxidsubstituierte Siloxane als Comonomere. Diese Verfahrensweise eröffnet die Möglicheit, bei Bedarf den Anteil sehr flexibler, hydrophober Strukturelemente weiter zu erhöhen.

Vorzugsweise stehen für die zum Ladungsausgleich notwendigen anorganischen oder organischen Anionen physiologisch vertretbare anorganische Reste, wie Chlorid, Bromid, Hydrogensulfat, Sulfat, bzw. organische Reste, wie Acetat, Propionat, Octanoat, Decanoat , Dodecanoat, Tetradecanoat, Hexadecanoat, Octadecanoat, Oleat, Citrat und Benzoat, Polyethercarboxylate, Polyethersulfate, Alkylsulfate, Alkylsulfonate und Arylsulfonate.

In analoger Weise erfolgt die Synthese der Aminoxidderivate. Hierzu werden die alpha, omega-polytertiäraminomodifizierten Polysiloxane mit anorganischen oder organischen Peroxiden umgesetzt. Die Durchführung von Aminoxidsynthesen ist allgemein bekannt (Houben/Weyl, Methoden der Organischen Chemie, Band XI/2, S.191-194 und Band E16a, S.408-412). Bevorzugte Peroxide sind z.B. Wasserstoffperoxid, Peressigsäure und Perbenzoesäure. Es ist möglich, die Peroxide 'in situ' aus entsprechenden Salzen, beispielsweise Percarbonaten und Perboraten, freizusetzen.
Es liegt im Rahmen der Erfindung, mehrere Siloxankomponenten unterschiedlicher Kettenlänge und/oder verschiedene sekundär-tertiäre Polyamine unter Beibehaltung der gewünschten Gesamtstöchiometrie zur Reaktion zu bringen. Es folgt hieraus z.B. die Möglichkeit, eine gewünschte Siloxankettenlänge durch Einsatz einer einzigen Siloxankomponente oder aber durch gezielte Mischung mehrerer Siloxankomponenten einzustellen. Analog dazu ist es möglich, eine vorteilhafte durchschnittliche Dichte an quaternierten Gruppen in alpha, omega-Stellung am Siloxan darzustellen.

Die Quaternierungsreaktionen und Aminoxidsynthesen werden bevorzugt in Wasser und/oder polaren organischen Lösungsmitteln ausgeführt. Geeignet sind z.B. Alkohole, speziell Methanol, Ethanol, i-Propanol und n-Butanol, Glycole, wie Ethylenglycol, Diethylenglycol, Triethylenglycol, die Methyl-, Ethyl- und Butylether der genannten Glycole, 1,2-Propylenglycol und 1,3-Propylenglycol, Ketone, wie Aceton und Methylethylketon, Ester, wie Ethylacetat, Butylacetat und 2-Ethylhexylacetat, Ether, wie Tetrahydrofuran und Nitroverbindungen, wie Nitromethan. Die Wahl des Lösungsmittels richtet sich wesentlich nach der Löslichkeit der Reaktionspartner und der angestrebten Reaktionstemperatur.

Die Reaktionen werden im Bereich von 20 °C bis 130 °C, vorzugsweise 40 °C bis 100 °C ausgeführt.

Die Erfindung betrifft weiterhin die Verwendung der erfindungsgemäßen Polyammonium-Polysiloxancopolymere sowie der Verbindungender Formeln (V) und (V') bzw. deren Säureadditionssalze in kosmetischen Formulierungen für die Haut-und Haarpflege.

Die Erfindung betrifft weiterhin die Verwendung der erfindungsgemäßen Polyammonium-Polysiloxancopolymere sowie der Verbindungender Formeln (V) und (V') bzw. deren Säureadditionssalze in Polituren für die Behandlung und Ausrüstung harter Oberflächen, in Formulierungen zum Trocknen von Automobilen und anderen harten Oberflächen nach maschinellen Wäschen.

Die Erfindung betrifft weiterhin die Verwendung der erfindungsgemäßen Polyammonium-Polysiloxancopolymere sowie der Verbindungender Formeln (V) und (V') bzw. deren Säureadditionssalze zur Ausrüstung von Textilien, Textilfasern, Papier und Holz.

Die Erfindung betrifft weiterhin die Verwendung der erfindungsgemäßen Polyammonium-Polysiloxancopolymere sowie der Verbindungender Formeln (V) und (V') bzw. deren Säureadditionssalze als Antistatikum und/oder als antibakterielles Mittel.

Die Erfindung betrifft weiterhin die Verwendung der erfindungsgemäßen Polyammonium-Polysiloxancopolymere sowie der Verbindungender Formeln (V) und (V') bzw. deren Säureadditionssalze als separate Weichmacher nach dem Waschen von Textilien mit auf anionischen und/oder nichtionogen Detergenzien beruhenden Formulierungen und als Weichmacher in auf anionischen und/oder nichtionogenen Tensiden beruhenden Formulierungen zur Textilwäsche.

Die Erfindung betrifft weiterhin die Verwendung der erfindungsgemäßen Polyammonium-Polysiloxancopolymere sowie der Verbindungender Formeln (V) und (V') bzw. deren Säureadditionssalze als Komponente zur Verbesserung des Haarglanzes in Shampoos, die auf anionischen, nichtionogenen, kationischen oder betainartigen Tensiden beruhen, in transparenten oder turbiden "Leave-on"- oder "Rinse-off"- Festigern, Schaumfestigern, Gelfestigern und Festigersprays.

Die Erfindung betrifft weiterhin die Verwendung der erfindungsgemäßen Polyammonium-Polysiloxancopolymere sowie der Verbindungender Formeln (V) und (V') bzw. deren Säureadditionssalze als Komponente als Hilfsmittel bei der Haarfärbung oder zur Retardierung des Ausblutens/Auswaschens von Farbstoffen aus gefärbtem Haar in Shampoos, die auf anionischen, nichtionogenen, kationischen oder betainartigen Tensiden beruhen, in transparenten oder turbiden "Leave-on"- oder "Rinse-off"-Festigern, Schaumfestigern, Gelfestigern und Festigersprays.

Die Erfindung betrifft weiterhin die Verwendung der erfindungsgemäßen Polyammonium-Polysiloxancopolymere sowie der Verbindungender Formeln (V) und (V') bzw. deren Säureadditionssalze als Komponente zur Verbesserung des Haarvolumens in Shampoos, die auf anionischen, nichtionogenen, kationischen oder betainartigen Tensiden beruhen, in transparenten oder turbiden "Leave-on"- oder "Rinse-off"- Festigern, Schaumfestigern, Gelfestigern und Festigersprays.

Die Erfindung betrifft weiterhin die Verwendung der erfindungsgemäßen Polyammonium-Polysiloxancopolymere sowie der Verbindungender Formeln (V) und (V') bzw. deren Säureadditionssalze als Komponente zur Reduktion der Kämmkräfte in Shampoos, die auf anionischen, nichtionogenen, kationischen oder betainartigen Tensiden beruhen, in transparenten oder turbiden "Leave-on"- oder "Rinse-off"-Festigern, Schaumfestigern, Gelfestigern und Festigersprays.

Die Erfindung betrifft weiterhin die Verwendung der erfindungsgemäßen Polyammonium-Polysiloxancopolymere sowie der Verbindungender Formeln (V) und (V') bzw. deren Säureadditionssalze als Komponente zur Verbesserung der Permanenz von Festigern in Shampoos, die auf anionischen, nichtionogenen, kationischen oder betainartigen Tensiden beruhen, in transparenten oder turbiden "Leave-on"- oder "Rinse-off"-Festigern, Schaumfestigern, Gelfestigern und Festigersprays.

Die Erfindung betrifft weiterhin Zusammensetzungen, enthaltend die erfindungsgemäßen Polyammonium-Polysiloxancopolymere und/oder die Verbindungen der Formeln (V) und (V') bzw. deren Säureadditionssalze zusammen mit mindestens einem weiteren für eine solche Zusammensetzung üblichen Inhaltsstoff, wie eine Waschmittelzusammensetzung, Politurzusammensetzung oder eine kosmetische Zusammensetzung.

### Beispielen

### Beispiel 1

116,3 g (0,0184 mol Epoxygruppen) eines Epoxysiloxans der durchschnittlichen Zusammensetzung 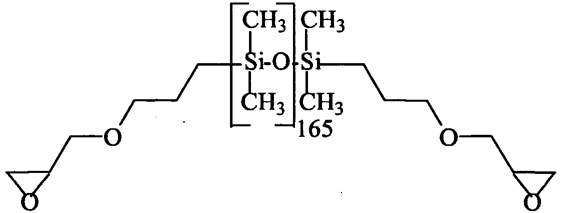 und 3,44 g (0,0184 mol) N,N,N',N'-Tetramethyldipropylentriamin werden in 120 g i-Propanol gelöst und für 8 Stunden auf Rückflußtemperatur erhitzt. Zum gebildeten hexatertiären Aminoderivat werden innerhalb von 10 Minuten 7,2g (0,0368 mol) einer 39%-igen Peressigsäure zugetropft und die Reaktion für 6 Stunden fortgesetzt. Im Verlauf der Reaktion nimmt die Lösung eine leicht rötliche Färbung an. Ein Test mit Kaliumjodid und Stärke zeigt an, daß nach Abschluß der Reaktion kein freies Peroxid mehr vorhanden ist. Nach Entfernung des Lösungsmittels im Vakuum werden 117,2 g einer viskosen Masse folgender Struktur erhalten: 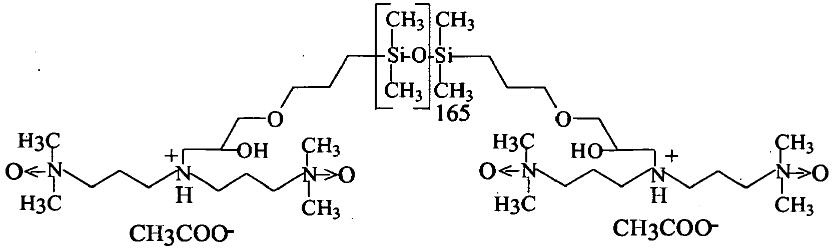 erhalten.

¹H-NMR für intermediär gebildetes hexatertiäres Aminoderivat

| **Substruktur** | **shift (ppm)** |
|---|---|
| -SiC**H**₂- | 0,45-0,5 (2H) |
| -CH(OH)C-**H**₂N[C**H**₂CH₂CH₂N(CH₃)₂]₂ | 2,3-2,5 (6H) |
| -CH(OH)CH₂N[CH₂CH₂C**H₂**N(CH₃)₂]₂ | 2,15-2,22 (4H) |
| -CH(OH)CH₂N[CH₂CH₂CH₂N(C**H₃**)₂]₂ | 2,1 (12H) |

¹H-NMR für Aminoxid

| **Substruktur** | **shift (ppm)** |
|---|---|
| -SiC**H₂**- | 0,4-0,5 (2H) |
| -CH(OH)CH₂N[CH₂CH₂CH₂NO(C**H₃**)₂]₂ | 3,17-3,27 (12H) |

### Beispiel 2

2a) 41,5 g (0,223 mol) Dodecanol werden unter Stickstoff bei Raumtemperatur vorgelegt. Unter intensiver Rührung werden innerhalb 10 Minuten 37,7 g (0,334 mol) Chloressigsäurechlorid zugetropft. Während des Zutropfens steigt die Temperatur auf 45 °C an und eine intensive HCl-Entwicklung setzt ein. Nach Beendigung des Zutropfens wird der Ansatz für 2 Stunden auf 100 °C erhitzt. Abschließend werden alle bis 100 °C/20 hPa siedenden Bestandteile abdestilliert. Es werden 57,8 g eines leicht gelb gefärbten, niedrig viskosen Chloressigsäureesters der Formel

   ClCH₂C(O)O(CH₂)₁₁CH₃

   erhalten.
   Die gaschromatographisch bestimmte Reinheit des Esters ist größer 99 %.
2b) 65,4 g (0,0104 mol Epoxygruppen) eines Epoxysiloxans der durchschnittlichen Zusammensetzung 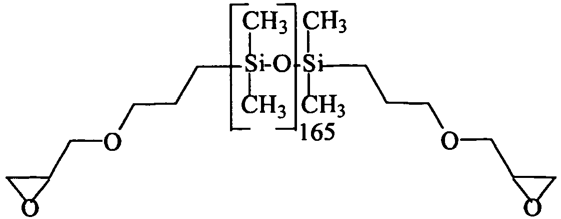 und 1,9 g (0,0104 mol) N,N,N',N'-Tetramethyldipropylentriamin werden in 72,7 g i-Propanol gelöst und für 8 Stunden auf Rückflußtemperatur erhitzt. Anschließend werden 5,4 g (0,0207 mol) des Chloressigsäureesters gemäß Beispiel 2a) zugesetzt und die Reaktion für 7 Stunden fortgesetzt. Nach Entfernen des Lösungsmittels werden 69,8 g einer gelblichen, viskosen Masse der Struktur 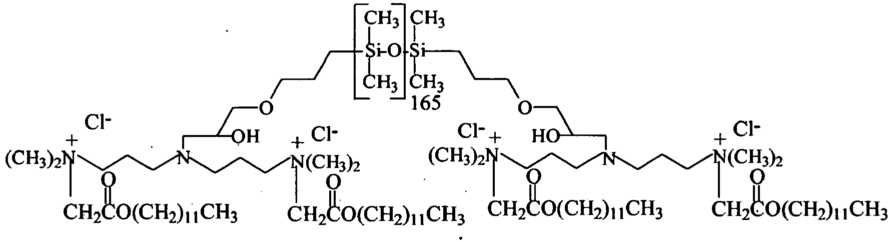 erhalten.

Gaschromatographisch wurde ein quantitativer Umsatz des Esters festgestellt.

¹H-NMR für quaternäres Material

| **Substruktur** | **shift (ppm)** |
|---|---|
| -SiC**H₂**- | 0,4-0,5 (2H) |
| -CH(OH)CH₂N[CH₂CH₂CH₂N⁺(CH₂-)(C**H₃**)₂]₂ | 3,35-3,51 (12H) |

### Beispiel 3

Zum Nachweis der weichmachenden Eigenschaften wurden gebleichte und an der Oberfläche nicht weiter ausgerüstete Baumwollstreifen einem Waschprozeß in Gegenwart von Ariel Futur^{®}, bentonithaltigem Dash 2 in 1^{®}, sowie des in Beispiel 2b beschriebenen polyquaternären Siloxans unterworfen. Es wurden folgende Randbedingungen eingehalten.

| | Streifen 1 | Streifen 2 | Streifen 3 |
|---|---|---|---|
| Streifengewicht (g) | 13,07 | 12,90 | 13,30 |
| Wassermenge (ml) | 653 | 645 | 666 |
| Detergenz (g) | 0,65 Ariel Futur^{®} | 0,64 Dash 2 in 1^{®} | 0,67 Ariel Futur^{®} |
| Esterquat Bsp. 2b (g) | 0,2 | - | - |
| Note Ø | 1,2 | 1,9 | 2,9 |

Das Wasser wird auf 60 °C erhitzt, die Detergenzien und im Falle des Baumwollstreifens 1 zusätzlich das polyquaternäre Siloxan gemäß Beispiel 2b gelöst. Anschließend werden die Baumwollstreifen in diesen Lösungen für 30 Minuten gewaschen. Nachfolgend werden die Streifen in 5x 600 ml Wasser gespült und abschließend 30 Minuten bei 120 °C getrocknet.

16 Testpersonen haben die drei Baumwollstreifen auf die Weichheit des Griffs hin bewertet, wobei die Note 1 dem weichesten Streifen und die Note 3 dem als am härtesten empfundenen Streifen zugeteilt wurde.

Im Ergebnis der Bewertung erhielt der Baumwollstreifen 1 die Durchschnittsnote 1,2. Der bentonitbehandelte Baumwollstreifen 2 wurde durchschnittlich mit 1,9 und der Streifen 3 mit 2,9 bewertet.

### Beispiel 4

4a) 238 g (2,24 mol) Diethylenglycol werden unter Stickstoff bei Raumtemperatur vorgelegt. Unter intensiver Rührung werden innerhalb einer Stunde 558 g (4,93 mol) Chloressigsäurechlorid zugetropft. Während des Zutropfens steigt die Temperatur auf 82 °C an und eine intensive HCl-Entwicklung setzt ein. Nach Beendigung des Zutropfens wird der Ansatz für 30 Minuten auf 130 °C erhitzt. Abschließend werden alle bis 130 °C/20 hPa siedenden Bestandteile abdestilliert. Es werden 566 g eines hellgelben Öls der Zusammensetzung

ClCH₂C(O)OCH₂CH₂OCH₂CH₂OC(O)CH₂Cl

erhalten.

Die gaschromatographisch bestimmte Reinheit des Esters beträgt 99,2 %.
¹³C-NMR:

| **Substruktur** | **Shift (ppm)** |
|---|---|
| Cl**C**H₂- | 40,7 |
| ClCH₂-**C**(O)- | 167,1 |
| ClCH₂-CH₂-C(O)-O**C**H₂- | 65,2 |
| ClCH₂-CH₂-C(O)-OCH₂**C**H₂- | 68,6 |

42,4 g (0,00151 mol) des quaternierten Siloxans gemäß Beispiel 2b und 1,59 g (0,00151 mol) des Diesters gemäß Beispiel 4a) werden in 45 g i-Propanol gelöst und für 8 Stunden auf Rückflußtemperatur erhitzt. Der klare Ansatz trübt sich bei Abkühlung nach Beendigung der Reaktion ein. Nach Entfernung des Lösungsmittels werden 39 g eines gelb-beigen viskosen Produktes mit Struktureinheiten 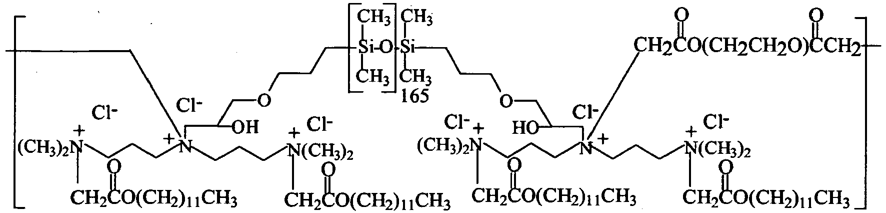 erhalten.
¹H-NMR

| **Substruktur** | **shift (ppm)** |
|---|---|
| -N⁺C**H₂**-C(O)O- | 4,07 |

### Beispiel 5

Zum Nachweis der weichmachenden Eigenschaften wurden gebleichte und an der Oberfläche nicht weiter ausgerüstete Baumwollstreifen einem Waschprozeß in Gegenwart von Ariel Futur^{®}, bentonithaltigem Dash 2 in 1^{®}, sowie des in Beispiel 4 beschriebenen polyquaternären Polysiloxans unterworfen. Es wurden folgende Randbedingungen eingehalten.

| | Streifen 1 | Streifen 2 | Streifen 3 |
|---|---|---|---|
| Streifengewicht (g) | 13,11 | 13,25 | 13,31 |
| Wassermenge (ml) | 654 | 664 | 667 |
| Detergenz (g) | 0,65 Ariel Futur^{®} | 0,66 Dash 2 in 1^{®} | 0,67 Ariel Futur^{®} |
| Esterquat Bsp. 4 (g) | 0,2 | - | - |
| Note Ø | 1,4 | 1,8 | 2,8 |

Das Wasser wird auf 60 °C erhitzt, die Detergenzien und im Falle des Baumwollstreifens 1 zusätzlich das polyquaternäre Polysiloxan gemäß Beispiel 4 gelöst. Anschließend werden die Baumwollstreifen in diesen Lösungen für 30 Minuten gewaschen. Nachfolgend werden die Streifen in 5x 600 ml Wasser gespült und abschließend 30 Minuten bei 120 °C getrocknet.

16 Testpersonen haben die drei Baumwollstreifen auf die Weichheit des Griffs hin bewertet, wobei die Note 1 dem weichesten Streifen und die Note 3 dem als am härtesten empfundenen Streifen zugeteilt wurde.

Im Ergebnis der Bewertung erhielt der Baumwollstreifen 1 die Durchschnittsnote 1,4. Der bentonitbehandelte Baumwollstreifen 2 wurde durchschnittlich mit 1,8 und der Streifen 3 mit 2,8 bewertet.

### Beispiel 6a

### Anionisches Shampoo

### (Zahlenwerte stellen Gew.-% dar)

| Erfindungsgemäße Komponente | 0,5 bis 5 |
|---|---|
| Ammonium Lauryl Sulfate* | 10.00 - 30.00 |
| Ammonium Laureth Sulfate* | 5.00 - 20.00 |
| Cocamidopropyl Betaine* | 0.00 - 15.00 |
| Lauramid DEA* | 0.00 - 5.00 |
| Cocamid Mea* | 0.00 - 5.00 |
| Dimethicone Copolyol* | 0.00 - 5.00 |
| Cyclopentasiloxane* | 0.00 - 5.00 |
| Quaternary Silicone* | 0.50 - 5.00 |
| Polyquaternium-10* | 0.00 - 2.00 |
| Preservative* | 0.00 - 0.50 |
| Fragrance* | 0.00 - 5.00 |
| Deionized Water* | q.s. 100% |
| Sodium Chloride* | q.s. |

| | |
|---|---|
| * per INCI-Namen | |

Diese Formulierung stellt eine Rahmenrezeptur dar. Formulierungen dieser Kategorie können die folgenden Komponenten, ohne auf diese beschränkt zu sein: Alkylsulfate, Alkylethersulfate, Natriumlaurylsulfat, Natriumlaurethsulfat, Ammoniumlaurylsulfat, Ammoniumlaurethsulfat, TEA-laurylsulfat, TEA-laurethsulfat, Alkylbenzolsulfonate, Alpha-olefinsulfonate, Paraffinsulfonate, Sulfosuccinate, N-Acyltauride, Sulfatierte Glyceride, Sulfatierte Alkanolamide, Carboxylatsalze, N-Acylaminosäuresalze, Silicone.

### Beispiel 6b

### Nichtionisches Shampoo

### (Zahlenwerte stellen Gew.-% dar)

| Erfindungsgemäße Komponente | 0,5 bis 5 |
|---|---|
| Lauramid DEA* | 10.00 - 30.00 |
| Lauramid Oxide* | 5.00 - 20.00 |
| Cocamid Mea* | 0.00 - 5.00 |
| Dimethicone Copolyol* | 0.00 - 5.00 |
| Quaternary Silicone* | 0.50 - 5.00 |
| Preservative* | 0.00 - 0.50 |
| Fragrance* | 0.00 - 5.00 |
| Deionized Water* | q.s. 100% |
| Sodium Chloride* | q.s. |

| | |
|---|---|
| * per INCI-Namen | |

Diese Formulierung stellt eine Rahmenrezeptur dar. Formulierungen dieser Kategorie enthalten die folgenden Komponenten, ohne auf diese beschränkt zu sein: Monoalkanolamide, Monoethanolamide, Monoisopropanolamide, Polyhydroxy-Derivate, Sucrosemonolaurat, Polyglycerolether, Aminoxide, polyethoxylierte Derivate, Sorbitan Derivate, Silicones.

### Beispiel 6c

### Amphoteres Shampoo

### (Zahlenwerte stellen Gew.-% dar)

| Erfindungsgemäße Komponente | 0,5 bis 5 |
|---|---|
| PEG-80 sorbitan laurate* | 10.00 - 30.00 |
| Lauroamphoglycinate* | 0.00 - 10.00 |
| Cocamidopropyl Hydroxysultaine* | 0.00 - 15.00 |
| PEG-150 distearate* | 0.00 - 5.00 |
| Laureth-13 carboxylate* | 0.00 - 5.00 |
| Quaternary Silicone* | 0.50 - 5.00 |
| Preservative* | 0.00 - 0.50 |
| Fragrance* | 0.00 - 5.00 |
| Deionized Water* | q.s. 100% |
| Sodium Chloride* | q.s. |

| | |
|---|---|
| *per INCI-Namen | |

Diese Formulierung ist eine Rahmenrezeptur. Formulierungen dieser Kategorie enthalten die folgenden Komponenten, ohne auf diese beschränkt zu sein: N-alkyl-α-iminodipropionate, N-alkyl-α-iminopropionate, Aminosäuren, Aminosäure -Derivate, Amidobetaine, Imidazolinium -Derivate, Sulfobetaine, Sultanine, Betaine, Silicones.

### Beispiel 6d

### Kationisches Shampoo

### (Zahlenwerte stellen Gew.-% dar)

| Erfindungsgemäße Komponente | 0,5 bis 5 |
|---|---|
| Laureth-13 carboxylate* | 10.00 - 30.00 |
| Isopropyl myristate* | 5.00 - 20.00 |
| Cocamidopropyl Betaine* | 0.00 - 15.00 |
| Lauramid DEA* | 0.00 - 5.00 |
| Cocamid Mea* | 0.00 - 5.00 |
| Quaternary Silicone* | 0.50 - 5.00 |
| Quaternary Silicone* | 0.50 - 5.00 |
| Preservative* | 0.00 - 0.50 |
| Fragrance* | 0.00 - 5.00 |
| Deionized Water* | q.s. 100% |
| Sodium Chloride* | q.s. |

| | |
|---|---|
| * per INCI-Namen | |

Diese Formulierung ist eine Rahmenrezeptur. Formulierungen dieser Kategorie enthalten die folgenden Komponenten, ohne auf diese beschränkt zu sein: bis-Quaternäre Ammonium Verbindungen, Bis-(trialkylammonioacetyl)-diamine, Amidoamine, Ammonioalkylester, Silicone.

### Beispiel 6e

### Festiger

### (Zahlenwerte stellen Gew.-% dar)

| Erfindungsgemäße Komponente | 0,5 bis 5 |
|---|---|
| Ceteareth-20* | 0.10 - 10.00 |
| Steareth-20* | 0.10 - 10.00 |
| Stearyl Alcohol* | 0.10 - 10.00 |
| Stearamidopropyl Dimethylamine* | 0.00 - 10.00 |
| Dicetyldimonium Chloride* | 0.00 - 10.00 |
| Quaternary Silicone* | 0.50 - 5.00 |
| Cyclopentasiloxane* | 0.00 - 5.00 |
| Dimethicone* | 0.00 - 5.00 |
| Preservative* | 0.00 - 0.50 |
| Fragrance* | 0.00 - 5.00 |
| Deionized Water* | q.s. 100% |

| | |
|---|---|
| * per INCI-Namen | |

Diese Formulierung ist eine Rahmenrezeptur. Formulierungen dieser Kategorie enthalten die folgenden Komponenten, ohne auf diese beschränkt zu sein: Fettsäuren, Fettsäureester, ethoxylierte Fettsäuren, Ethoxylierte Fettsäureester, Fettalkohole, Ethoxylierte Fettalkhole, Glykol, Glykolester, Glycerol , Glycerolester, Lanolin, Lanolin-Derivate, Mineralöl, Petrolatum, Lecithin, Lecithin-Derivate, Wachse, Wachs-Derivate, kationische Polymere, Proteine, Protein-Derivate, Aminosäuren, Aminosäure-Derivate, Feuchthaltemittel, Verdickungsmittel, Silicone.

### Beispiel 6f

### "Clear Rinse-Off"- Festiger

### (Zahlenwerte stellen Gew.-% dar)

| Erfindungsgemäße Komponente | 0,5 bis 5 |
|---|---|
| Glycerin* | 0.10 - 10.00 |
| Cetrimonium Chloride* | 0.00 - 10.00 |
| Quaternary Silicone* | 0.50 - 5.00 |
| Hydroxyethylcellulose* | 0.00 - 5.00 |
| Preservative* | 0.00 - 0.50 |
| Fragrance* | 0.00 - 5.00 |
| Deionized Water* | q.s. 100% |

| | |
|---|---|
| *per INCI-Namen | |

Diese Formulierung ist eine Rahmenrezeptur. Formulierungen dieser Kategorie enthalten die folgenden Komponenten, ohne auf diese beschränkt zu sein: Fettsäuren, Fettsäureester, Ethoxylierte Fettsäuren, Ethoxylierte Fettsäureester, Fettalkohole, Ethoxylierte Fettalkhole, Glykol, Glykolester, Glycerol , Glycerolester, Lanolin, Lanolin-Derivate, Mineralöl, Petrolatum, Lecithin, Lecithin-Derivate, Wachse, Wachs -Derivate, kationische Polymere, Proteine, Protein-Derivate, Aminosäuren, Aminosäure-Derivate, Feuchthaltmittel, Verdickungsmittel, Silicone.

### Beispiel 6g

### Schaumfestiger für Haare

### (Zahlenwerte stellen Gew.-% dar)

| Erfindungsgemäße Komponente | 0,5 bis 5 |
|---|---|
| Quaternary Silicone* | 0.50 - 5.00 |
| Nonoxynol-15* | 0.00 - 2.00 |
| Nonoxynol-20* | 0.00 - 2.00 |
| Fragrance* | 0.00 - 5.00 |
| Propellant* | 0.00 - 20.00 |
| Preservative* | 0.00 - 0.50 |
| Fragrance* | 0.00 - 5.00 |
| Deionized Water* | q.s. 100% |

| | |
|---|---|
| * per INCI-Namen | |

Diese Formulierung ist eine Rahmenrezeptur. Formulierungen dieser Kategorie enthalten die folgenden Komponenten, ohne auf diese beschränkt zu sein:
Fettsäuren, Fettsäureester, Ethoxylierte Fettsäuren, Ethoxylierte Fettsäureester, Fett alkohole, Ethoxylierte Fettalkhole, Glykol, Glykolester, Glycerol , Glycerolester, Lanolin, Lanolin-Derivate, Mineralöl, Petrolatum, Lecithin, Lecithin-Derivate, Wachse, Wachs -Derivate, kationische Polymere, Proteine, Protein-Derivate, Aminosäuren, Aminosäure-Derivate, Feuchthaltmittel, Verdickungsmittel, Silicone, Lösemittel, Ethanol, Isopropanol, Isoparafinische Lösemittel, Butan, Propan, Isobutan, FCKW, Fluorierte Treibmittel, Dimethylether, komprimierte Gase.

### Beispiel 6h

### Pumpspray (Festiger) für Haare

### (Zahlenwerte stellen Gew.-% dar)

| Erfindungsgemäße Komponente | 0,5 bis 5 |
|---|---|
| Quaternary Silicone* | 0.50 - 5.00 |
| Cyclomethicone* | 0.00 - 80.00 |
| Ethanol* | 0.00 - 80.00 |
| Preservative* | 0.00 - 0.50 |
| Fragrance* | 0.00 - 5.00 |
| Deionized Water* | q.s. 100% |

| | |
|---|---|
| * per INCI-Namen | |

Diese Formulierung ist eine Rahmenrezeptur. Formulierungen dieser Kategorie enthalten die folgenden Komponenten, ohne auf diese beschränkt zu sein: Fettsäuren, Fettsäureester, Ethoxylierte Fettsäuren, Ethoxylierte Fettsäureester, Fettalkohole, Ethoxylierte Fettalkhole, Glykol, Glykolester, Glycerol , Glycerolester, Lanolin, Lanolin-Derivate, Mineralöl, Petrolatum, Lecithin, Lecithin-Derivate, Wachse, Wachs-Derivate, kationische Polymere, Proteine, Protein-Derivate, Aminosäuren, Aminosäure-Derivate, Feuchthaltmittel, Verdickungsmittel, Silicone, Lösemittel, Ethanol, Isopropanol, Isoparafinische Lösemittel, Butan, Propan, Isobutan, FCKW, Fluorierte Treibmittel, Dimethylether, komprimierte Gase.

### Beispiel 6i

### Festigerspray für Haare

### (Zahlenwerte stellen Gew.-% dar)

| Erfindungsgemäße Komponente | 0,5 bis 5 |
|---|---|
| Quaternary Silicone* | 0.50 - 5.00 |
| Cyclomethicone* | 0.00 - 80.00 |
| Ethanol* | 0.00 - 50.00 |
| Propellant* | 0.00 - 50.00 |
| Preservative* | 0.00 - 0.50 |
| Fragrance* | 0.00 - 5.00 |
| Deionized Water* | q.s. 100%. |

| | |
|---|---|
| *per INCI-Namen | |

Diese Formulierung ist eine Rahmenrezeptur. Formulierungen dieser Kategorie enthalten die folgenden Komponenten, ohne auf diese beschränkt zu sein: Fettsäuren, Fettsäureester, ethoxylierte Fettsäuren, ethoxylierte Fettsäureester, Fettalkohole, ethoxylierte Fettalkhole, Glykol, Glykolester, Glycerol , Glycerolester, Lanolin, Lanolin-Derivate, Mineralöl, Petrolatum, Lecithin, Lecithin-Derivate, Wachse, Wachs -Derivate, kationische Polymere, Proteine, Protein-Derivate, Aminosäuren, Aminosäure-Derivate, Feuchthaltmittel, Verdickungsmittel, Silicone, Lösemittel, Ethanol, Isopropanol, Isoparafinische Lösemittel, Butan, Propan, Isobutan, FCKW, Fluorierte Treibmittel, Dimethylether, komprimierte Gase.

### Beispiel 6j

### Gelfestiger für Haare

### (Zahlenwerte stellen Gew.-% dar)

| Erfindungsgemäße Komponente | 0,5 bis 5 |
|---|---|
| Quaternary Silicone* | 0.50 - 5.00 |
| Hyrdoxyethylcellulose* | 0.00 - 2.00 |
| Fragrance* | 0.00 - 5.00 |
| Preservative* | 0.00 - 0.50 |
| Citric acid* | 0.00 - 2.00 |
| Deionized Water* | q.s. 100% |

| | |
|---|---|
| *per INCI-Namen | |

Diese Formulierung ist eine Rahmenrezeptur. Formulierungen dieser Kategorie enthalten die folgenden Komponenten, ohne auf diese beschränkt zu sein:
Verdickungsmittel, Cellulose-Derivate, Acrylsäure-Derivate, Fixativpolymere, Conditioner-Chemikalien, Glykol, Glykolester, Glycerol, Glycerolester, Lanolin, Lanolin -Derivate, Mineralöl, Petrolatum, Lecithin, Lecithin-Derivate, Wachse, Wachs -Derivate, kationische Polymere, Proteine, Protein-Derivate, Aminosäuren, Aminosäure-Derivate, Feuchtemittel, Silicone, Lösemittel, Ethanol, Isopropanol, Isoparafinische Lösemittel.

### Beispiel 6k

### Styling Gel für Haare

### (Zahlenwerte stellen Gew.-% dar)

| Erfindungsgemäße Komponente | 0,5 bis 5 |
|---|---|
| Quaternary Silicone* | 0.50 - 5.00 |
| Fixative* | 0.10-10.00 |
| Hyrdoxyethylcellulose* | 0.00 - 2.00 |
| Fragrance* | 0.00 - 5.00 |
| Fragrance* | 0.00 - 5.00 |
| Citric acid* | 0.00 - 2.00 |
| Deionized Water* | q.s. 100% |

| | |
|---|---|
| *per INCI-Namen | |

Diese Formulierung ist eine Rahmenrezeptur. Formulierungen dieser Kategorie enthalten die folgenden Komponenten, ohne auf diese beschränkt zu sein:
Fixativpolymere, Lacke, Verdickungsmittel, Cellulose-Derivate, Acrylsäure-Derivate, Conditioner-Chemikalien, Glykol, Glykolester, Glycerol , Glycerolester, Lanolin, Lanolin-Derivate, Mineralöl, Petrolatum, Lecithin, Lecithin-Derivate, Wachse, Wachs-Derivate, kationische Polymere, Proteine, Protein-Derivate, Aminosäuren, Aminosäure-Derivate, Feuchtemittel, Silicone, Lösemittel, Ethanol, Isopropanol, Isoparafinische Lösemittel.

### Beispiel 61

### Styling Spray für Haare

### (Zahlenwerte stellen Gew.-% dar)

| Erfindungsgemäße Komponente | 0,5 bis 5 |
|---|---|
| Quaternary Silicone* | 0.50 - 5.00 |
| Cyclomethicone* | 0.00 - 80.00 |
| Fixative* | 0.10 - 10.00 |
| Ethanol* | 0.00 - 50.00 |
| Propellant* | 0.00 - 50.00 |
| Preservative* | 0.00 - 0.50 |
| Fragrance* | 0.00 - 5.00 |
| Deionized Water* | q.s. 100% |

| | |
|---|---|
| *per INCI-Namen | |

Diese Formulierung ist eine Rahmenrezeptur. Formulierungen dieser Kategorie enthalten die folgenden Komponenten, ohne auf diese beschränkt zu sein: Fixativpolymere, Lacke, Verdickungsmittel, Cellulose-Derivate, Acrylsäure-Derivate, Conditioner-Chemikalien, Glykol, Glykolester, Glycerol , Glycerolester, Lanolin, Lanolin-Derivate, Mineralöl, Petrolatum, Lecithin, Lecithin-Derivate, Wachse, Wachs-Derivate, kationische Polymere, Proteine, Protein-Derivate, Aminosäuren, Aminosäure-Derivate, Feuchtemittel, Silicone, Lösemittel, Ethanol, Isopropanol, Isoparafinische Lösemittel, Butan, Propan, Isobutan, FCKW, fluorierte Treibmittel, Dimethylether, komprimierte Gase.

### Beispiel 6m

### Pumpspray (Styling) für Haare

### (Zahlenwerte stellen Gew.-% dar)

| Erfindungsgemäße Komponente | 0,5 bis 5 |
|---|---|
| Quaternary Silicone* | 0.50 - 5.00 |
| Fixative* | 0.10-10.00 |
| Cyclomethicone* | 0.00 - 80.00 |
| Ethanol* | 0.00 - 50.00 |
| Preservative* | 0.00 - 0.50 |
| Fragrance* | 0.00 - 5.00 |
| Deionized Water* | q.s. 100% |

| | |
|---|---|
| *per INCI-Namen | |

Diese Formulierung ist eine Rahmenrezeptur. Formulierungen dieser Kategorie enthalten die folgenden Komponenten, ohne auf diese beschränkt zu sein: Vinyl-Derivate, Fixativpolymere, Lacke, Verdickungsmittel, Fettsäuren, Fettsäureester, ethoxylierte Fettsäuren, ethoxylierte Fettsäureester, Fettalkohole, ethoxylierte Fettalkhole Cellulose-Derivate, Acrylsäure-Derivate, Conditioner-Chemikalien, Glykol, Glykolester, Glycerol, Glycerolester, Lanolin, Lanolin - Derivate, Mineralöl, Petrolatum, Lecithin, Lecithin-Derivate, Wachse, Wachs - Derivate, kationische Polymere, Proteine, Protein -Derivate, Aminosäuren, Aminosäure-Derivate, Feuchtemittel, Silicone, Lösemittel, Ethanol, Isopropanol, Isoparafinische Lösemittel, Butan, Propan, Isobutan, FCKW, fluorierte Treibmittel, Dimethylether, komprimierte Gase.

Die positive Beeinflussung folgender Effekte kann von den erfindungsgemäßen Siloxanderivaten bei Anwendung im Haarkosmetikbereich erwartet werden:

| | |
|---|---|
| **1** | **Festigung** |
| **2** | **Glanz** |
| **3** | **Fixierung (Halt)** |
| **4** | **Körper** |
| **5** | **Volumen** |
| **6** | **Feuchtigkeitsregulierung** |
| **7** | **Farbretention** |
| **8** | **Schutz vor Umwelteinflüssen (UV, Salzwasser u.s.w.)** |
| **9** | **Wiederformbarkeit** |
| **10** | **Antistatische Eigenschaften** |
| **11** | **Färbbarkeit** |

## Patentansprüche

1. Polyammonium-Polysiloxancopolymere; enthaltend Wiederholungseinheiten der Formel (I): 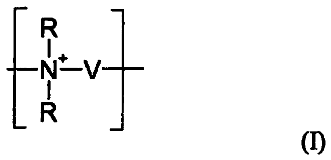 worin R jeweils unabhängig voneinander organische Gruppen sind, die jeweils mindestens eine Gruppe enthalten, die aus quaternären Ammoniumgruppen oder Aminoxidgruppen ausgewählt sind,
V ausgewählt wird aus der Gruppe V¹ und der Gruppe V²,
worin
V² ausgewählt wird aus zweiwertigen oder dreiwertigen, geradkettigen, cyclischen oder verzweigten, gesättigten, ungesättigten oder aromatischen Kohlenwasserstoffresten mit bis zu 1000 Kohlenstoffatomen (wobei die Kohlenstoffatome des unten definierten Polysiloxanrestes Z² nicht mitgezählt werden), die gegebenenfalls eine oder mehrere Gruppen, ausgewählt aus
-O-,
-NR²-,
-N⁺R²₂-,
worin R² Wasserstoff, einen einwertigen, geradkettigen, cyclischen oder verzweigten, gesättigten, ungesättigten oder aromatischen Kohlenwasserstoffrest mit bis zu 100 Kohlenstoffatomen darstellt, der eine oder mehrere Gruppen ausgewählt aus -O-, -NH-, -C(O)- und -C(S)- enthalten kann, und der gegebenenfalls durch eine oder mehrere Substituenten, ausgewählt aus der Gruppe, die besteht aus einer Hydroxylgruppe, einer gegebenenfalls substituierten, bevorzugt ein oder mehrere Stickstoffatome enthaltenden heterocyclischen Gruppe, Amino, Alkylamino, Dialkylamino, Polyetherresten und Polyetheresterresten substituiert sein kann, wobei wenn mehrere Gruppen -NR²- vorliegen, diese gleich oder verschieden sein können,
-C(O)-,
-C(S)-
enthalten kann, und
der Rest V² gegebenenfalls durch eine oder mehrere Hydroxylgruppen substituiert sein kann, und
der Rest V² mindestens eine Gruppe -Z²- der Formel 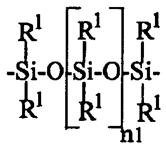 enthält, worin
R¹ gleich oder verschieden sein kann und aus der Gruppe ausgewählt wird, die besteht aus: C₁ bis C₂₂ Alkyl, Fluor(C₁-C₁₀)alkyl und C₆-C₁₀ Aryl, und
n₁ = 20 bis 1000 bedeutet,
V¹ ausgewählt wird aus zweiwertigen oder dreiwertigen, geradkettigen, cyclischen oder verzweigten, gesättigten, ungesättigten oder aromatischen Kohlenwasserstoffresten mit bis zu 1000 Kohlenstoffatomen, die gegebenenfalls eine oder mehrere Gruppen, ausgewählt aus
-O-,
-NR²-, -N⁺R²₂-,
worin R² wie oben definiert ist, und wobei die Gruppen R² in den Gruppen V¹ und V² gleich oder verschieden sein können,
-C(O)-,
-C(S)- und
-Z¹- enthalten kann, worin -Z¹- eine Gruppe der Formel 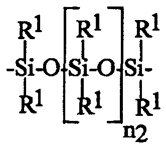 ist, worin
R¹ wie oben definiert ist, wobei die Gruppen R¹ in den Gruppen V¹ und V² gleich oder verschieden sein können, und
n₂ = 0 bis 19 bedeutet,
und der Rest V¹ gegebenenfalls durch eine oder mehrere Hydroxylgruppen substituiert sein kann,
worin die Gruppen V¹ und V² in den Polyammonium-Polysiloxancopolymeren gleich oder verschieden sein können, mit der Maßgabe, dass wenigstens eine Gruppe Z¹ oder Z² vorhanden ist, und
worin die aus den Ammoniumgruppen resultierenden positiven Ladungen durch organische oder anorganische Säureanionen neutralisiert sind.

2. Polyammonium-Polysiloxancopolymere nach Anspruch 1, worin R jeweils mindestens eine Gruppe der Formel (II) ist: 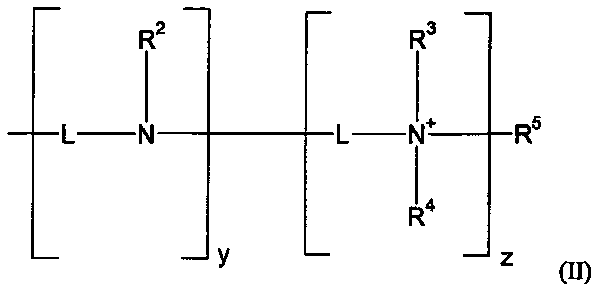 worin
L jeweils unabhängig voneinander ein zweiwertiger geradkettiger, cyclischer oder verzweigter, gesättigter, ungesättigter oder aromatischer Kohlenwasserstoffrest mit bis zu 30 Kohlenstoffatomen darstellt, der eine oder mehrere Gruppen ausgewählt aus -O-, -NH-, -C(O)- und -C(S)- enthalten kann, und der gegebenenfalls durch eine oder mehrere Substituenten, ausgewählt aus der Gruppe, die besteht aus einer Hydroxylgruppe, einer Carboxylgruppe, einer Carboxylatgruppe, einer gegebenenfalls substituierten, bevorzugt ein oder mehrere Stickstoffatome enthaltenden heterocyclischen Gruppe, Amino, Alkylamino, Dialkylamino, Ammoniumgruppe, Polyetherresten und Polyetheresterresten substituiert sein kann,
R² wie oben definiert ist,
R³, R⁴ und R⁵ jeweils unabhängig voneinander ausgewählt sind aus einwertigen, geradkettigen, cyclischen oder verzweigten, gesättigten, ungesättigten oder aromatischen Kohlenwasserstoffresten mit bis zu 30 Kohlenstoffatomen, die eine oder mehrere Gruppen ausgewählt aus -O-, -NH-, -C(O)- und -C(S)- enthalten können, und gegebenenfalls durch einen oder mehrere Substituenten, ausgewählt aus der Gruppe, die besteht aus einer Hydroxylgruppe, einer Carboxylgruppe, einer Carboxylatgruppe, einer gegebenenfalls substituierten, bevorzugt ein oder mehrere Stickstoffatome enthaltenden heterocyclischen Gruppe, Amino, Alkylamino, Dialkylamino, Ammoniumgruppe, Polyetherresten und Polyetheresterresten substituiert sein können, und
R³ oder R⁴ Sauerstoff sein können unter Bildung eines Aminoxids, und
z von 1 bis 10 ist und y von 0 bis 10 sein kann.

3. Polyammonium-Polysiloxancopolymere nach Anspruch 1 oder 2, worin R jeweils eine Gruppe der Formel (III) ist 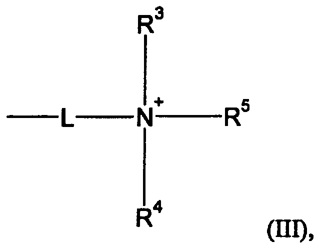 worin L, R³, R⁴ und R⁵ wie oben definiert sind.

4. Polyammonium-Polysiloxancopolymere nach einem der Ansprüche 1 bis 3, worin R jeweils mindestens eine Aminoxid-Gruppe der Formel (IV) ist: 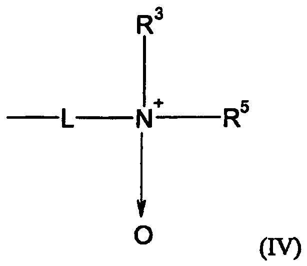 worin L, R³ und R⁵ wie oben definiert sind.

5. Polyammonium-Polysiloxancopolymere nach einem der Ansprüche 2 bis 4, worin L ausgewählt wird aus C₁ bis C₁₀-Alkylengruppen, die linear, verzweigt, oder cyclisch sein können.

6. Polyammonium-Polysiloxancopolymere nach einem der Ansprüche 1 bis 5, **dadurch gekennzeichnet, dass** sie mindestens zwei Wiederholungseinheiten V aufweist.

7. Polyammonium-Polysiloxancopolymere nach einem der Ansprüche 1 bis 5, **dadurch gekennzeichnet, dass** sie mindestens zwei Wiederholungseinheiten V² aufweist.

8. Polyammonium-Polysiloxancopolymere nach einem der Ansprüche 1 bis 7, worin V² eine Gruppe der folgenden Formel
-V²*-Z²-V²*-
ist, worin Z² wie oben definiert ist und V²* einen zweiwertigen geradkettigen, cyclischen oder verzweigten, gesättigten, ungesättigten oder aromatischen Kohlenwasserstoffrest mit bis zu 40 Kohlenstoffatomen darstellt, der gegebenenfalls eine oder mehrere Gruppen, ausgewählt aus -O-, -NR²-, -N⁺R²₂-, worin R² wie oben definiert ist, -C(O)- und -C(S)- enthalten kann, und der Rest V²* gegebenenfalls durch eine oder mehrere Hydroxylgruppen substituiert sein kann.

9. Polyammonium-Polysiloxancopolymere nach einem der Ansprüche 1 bis 8, worin die Gruppe V¹ ausgewählt wird aus zweiwertigen, geradkettigen, cyclischen oder verzweigten, gesättigten, ungesättigten oder aromatischen Kohlenwasserstoffresten mit bis zu 600 Kohlenstoffatomen, die gegebenenfalls eine oder mehrere Gruppen, ausgewählt aus -O-, -NR²-, -N⁺R²₂-, worin R² wie oben definiert ist, -C(O)-, -C(S)- und -Z¹- enthalten können, worin -Z¹- eine Gruppe der Formel 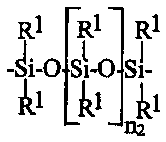 ist, worin
R¹ C₁ bis C₃-Alkyl, Fluor(C₃-C₆)-alkyl oder C₆-Aryl ist, und
n₂ wie oben definiert ist.

10. Verbindung der Formel (V): 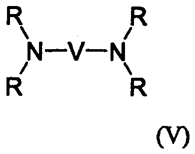 worin R und V wie oben definiert sind, mit der Maßgabe, dass V Z¹ oder Z² enthält, oder ein Säureadditionssalz davon.

11. Verbindung der Formel (V'): 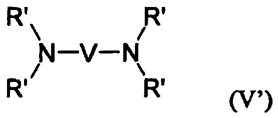 worin R' jeweils eine organische Gruppe ist, die jeweils mindestens eine tertiäre Aminogruppe enthält, und V wie oben defniert ist, mit der Maßgabe, dass V Z¹ oder Z² enthält, oder ein Säureadditionssalz davon.

12. Verbindung nach Anspruch 11, worin R' jeweils eine Gruppe der folgenden Formel darstellt: 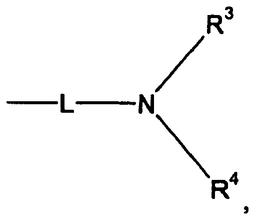 worin R³ und R⁴ eine Methyl- oder Ethylgruppe darstellt, und L eine C2-C6-Alkylengruppe darstellt.

13. Verbindung nach Anspruch 10, 11 oder 12, worin V V² ist.

14. Verfahren zur Herstellung der Polyammonium-Polysiloxancopolymere nach einem der Ansprüche 1 bis 9, worin die Verbindung der Formel (V) mit mindestens einem bifunktionellen Quaternierungsmittel umgesetzt wird.

15. Verfahren nach Anspruch 14, worin die bifunktionellen Quaternierungsmittel ausgewählt werden aus Verbindungen, die zwei funktionelle Gruppen aufweisen, die aus der Gruppe ausgewählt werden, die aus Epoxygruppen und Bishalogenalkylgruppen besteht.

16. Verwendung der Polyammonium-Polysiloxancopolymere nach einem der Ansprüche 1 bis 9 sowie der Verbindungen nach einem der Ansprüche 10 bis 13 in kosmetischen Formulierungen für die Haut- und Haarpflege.

17. Verwendung der Polyammonium-Polysiloxancopolymere nach einem der Ansprüche 1 bis 9 sowie der Verbindungen nach einem der Ansprüche 10 bis 13 in Polituren für die Behandlung und Ausrüstung harter Oberflächen, in Formulierungen zum Trocknen von Automobilen und anderen harten Oberflächen nach maschinellen Wäschen.

18. Verwendung der Polyammonium-Polysiloxancopolymere nach einem der Ansprüche 1 bis 9 sowie der Verbindungen nach einem der Ansprüche 10 bis 13 zur Ausrüstung bzw. Behandlung von Textilien, Textilfasern, Papier und Holz.

19. Verwendung der Polyammonium-Polysiloxancopolymere nach einem der Ansprüche 1 bis 9 sowie der Verbindungen nach einem der Ansprüche 10 bis 13 als Antistatikum und/oder antibakterielles Mittel.

20. Verwendung der Polyammonium-Polysiloxancopolymere nach einem der Ansprüche 1 bis 9 sowie der Verbindungen nach einem der Ansprüche 10 bis 13 als separate Weichmacher nach dem Waschen von Textilien mit auf anionischen und/oder nichtionogen Detergenzien beruhenden Formulierungen und als Weichmacher in auf anionischen und/oder nichtionogenen Tensiden beruhenden Formulierungen zur Textilwäsche.

21. Verwendung der Polyammonium-Polysiloxancopolymere nach einem der Ansprüche 1 bis 9 sowie der Verbindungen nach einem der Ansprüche 10 bis 13 als Komponente zur Verbesserung des Haarglanzes in Shampoos, die auf anionischen, nichtionogenen, kationischen oder betainartigen Tensiden beruhen, in transparenten oder turbiden "Leave-on"- oder "Rinse-off"-Festigern, Schaumfestigern, Gelfestigern und Festigersprays.

22. Verwendung der Polyammonium-Polysiloxancopolymere nach einem der Ansprüche 1 bis 9 sowie der Verbindungen nach einem der Ansprüche 10 bis 13 als Komponente als Hilfsmittel bei der Haarfärbung oder zur Retardierung des Ausblutens/Auswaschens von Farbstoffen aus gefärbtem Haar in Shampoos, die auf anionischen, nichtionogenen, kationischen oder betainartigen Tensiden beruhen, in transparenten oder turbiden "Leave-on"-oder "Rinse-off"-Festigern, Schaumfestigern, Gelfestigern und Festigersprays.

23. Verwendung der Polyammonium-Polysiloxancopolymere nach einem der Ansprüche 1 bis 9 sowie der Verbindungen nach einem der Ansprüche 10 bis 13 als Komponente zur Verbesserung des Haarvolumens in Shampoos, die auf anionischen, nichtionogenen, kationischen oder betainartigen Tensiden beruhen, in transparenten oder turbiden "Leave-on"- oder "Rinse-off"-Festigern, Schaumfestigern, Gelfestigern und Festigersprays.

24. Verwendung der Polyammonium-Polysiloxancopolymere nach einem der Ansprüche 1 bis 9 sowie der Verbindungen nach einem der Ansprüche 10 bis 13 als Komponente zur Reduktion der Kämmkräfte in Shampoos, die auf anionischen, nichtionogenen, kationischen oder betainartigen Tensiden beruhen, in transparenten oder turbiden "Leave-on"- oder "Rinse-off"-Festigern, Schaumfestigern, Gelfestigern und Festigersprays.

25. Verwendung der Polyammonium-Polysiloxancopolymere nach einem der Ansprüche 1 bis 9 sowie der Verbindungen nach einem der Ansprüche 10 bis 13, als Komponente zur Verbesserung der Permanenz von Festigern in Shampoos, die auf anionischen, nichtionogenen, kationischen oder betainarkigen Tensiden beruhen, in transparenten oder turbiden "Leave-on"-oder "Rinse-off"- Festigern, Schaumfestigern, Gelfestigern und Festigersprays.

26. Zusammensetzungen, enthaltend mindestens ein Polyammonium-Polysiloxancopolymer nach einem der Ansprüche 1 bis 9 oder eine Verbindung nach einem der Ansprüche 10 bis 13 zusammen mit mindestens einem weiteren für die Zusammensetzung üblichen Inhaltsstoff.

27. Zusammensetzung nach Anspruch 26, die eine Waschmittelzusammensetzung, Politurzusammensetzung oder eine kosmetische Zusammensetzung ist.

## Claims

1. Polyammonium polysiloxane copolymers, containing repeating units of the following formula (I): 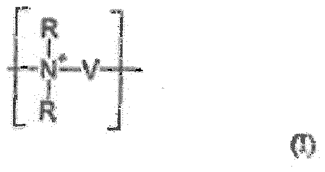 wherein R are each independently organic groups, each containing at least one group selected from quaternary ammonium groups and amine oxide groups,
V is selected from the group V¹ and the group V²
wherein
V² Is selected from divalent or trivalent, linear, cyclic or branched, saturated, unsaturated or aromatic hydrocarbon radicals having up to 1000 carbon atoms (whereby the carbon atoms of the polysiloxane radical Z² defined below are not included) which optionally contain one or more groups selected from
-O-,
-NR²-,
-N⁺R²₂-,
wherein R² represents hydrogen, a monovalent straight, cyclic or branched, saturated, unsaturated or aromatic hydrocarbon radical having up to 100 carbon atoms, which may contain one or more groups selected from -O-, -NH-, -C(O)- and -C(S)-, and which Is optionally substituted by one or more substituents selected from the group consisting of a hydroxyl group, an optionally substituted heterocyclic group which preferably contains one or more nitrogen atoms, amino, alkylamino, dialkylamino, polyether radicals, polyetherester radicals, wherein, provided multiple groups -NR²- are present, these may be the same or different,
-C(O)-,
-C(S)-,
and
the radical V² may be optionally substituted by one or more hydroxyl groups, and
the radical V² comprises at least one group -Z²- of the following formula 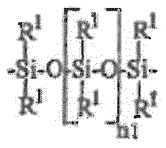 wherein
R¹ may be the same or different and is selected from the group consisting of C₁-C₂₂ alkyl, fluoro(C₁-C₁₀) alkyl and C₆-C₁₀ aryl, and
n₁ = 20 to 1000,
V¹ Is selected from divalent or trivalent, linear, cyclic or branched, saturated, unsaturated or aromatic hydrocarbon radicals having up to 1000 carbon atoms which optionally contain one or more groups selected from
-O-,
-NR²-,
-N⁺R²₂-
wherein R² is as defined above and wherein the groups R² in the groups V¹ and V² may be the same or different,
-C(O)-,
-C(S)- and
-Z¹-, wherein -Z¹- represents a group of the following formula 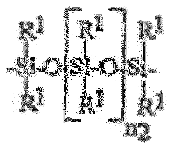 wherein
R¹ is as defined above, wherein the groups R¹ in the groups V¹ and V² may be the same or different, and
n₂ = 0 to 19,
and the radical V¹ is optionally substituted by one or more hydroxyl groups
wherein the groups V¹ and V² in the polyammonium polysiloxane copolymers can be the same or different, with the proviso that at least one group Z¹ or Z² is present and
wherein the resulting positive charge from the ammonium groups is neutralized by organic or inorganic acid anions.

2. Polyammonium polysiloxane copolymers according to claim 1, wherein R each is at least one group of the following formula (II): 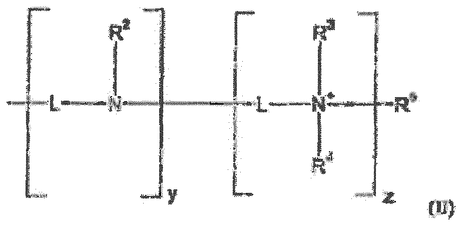 wherein
L each independently represents a divalent linear, cyclic or branched, saturated, unsaturated or aromatic hydrocarbon radical having up to 30 carbon atoms, which may contain one or more groups selected from -O-, -NH-, -C(O)- and -C(S)-, and which is optionally substituted by one or more substituents selected from the group consisting of a hydroxyl group, a carboxyl group, a carboxylate group, an optionally substituted heterocyclic group which preferably contains one or more nitrogen atoms, amino, alkylamino, dialkylamino, ammonium group, polyether radicals and polyetherester radicals,
R² is as defined above,
R³, R⁴ and R⁵ are each Independently selected from monovalent linear, cyclic or branched, saturated, unsaturated or aromatic hydrocarbon radicals having up to 30 carbon atoms which may contain one or more groups selected from -O-, -NH-, -C(O)- and -C(S)-, and which is optionally substituted by one or more substituents selected from the group consisting of a hydroxyl group, a carboxyl group, a carboxylate group, an optionally substituted heterocyclic group which preferably contains one or more nitrogen atoms, amino, amino, alkylamino, dialkylamino, ammonium group, polyether radicals and polyetherester radicals, and
R³ or R⁴ may be oxygen while forming an amine oxide, and
z is from 1 to 10 and y may be from 0 to 10.

3. Polyammonium polysiloxane copolymers according to claim 1 or 2, wherein R Is each a group of the following formula (III) 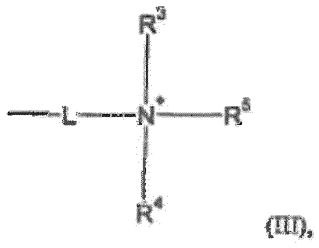 wherein L, R³, R⁴ and R⁵ are as defined above.

4. Polyammonlum polysiloxane copolymers according to one of claims 1 to 3, wherein R each is at least one amine oxide group of the following formula (IV): 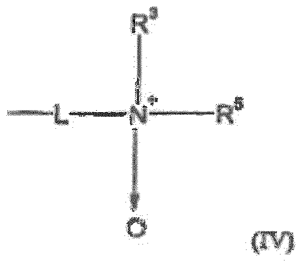 wherein L, R³ and R⁵ are as defined above.

5. Polyammonium polysiloxane copolymers according to one of claims 2 to 4 wherein L is selected from C₁ to C₁₀ alkylene groups, which may be linear, branched or cyclic.

6. Polyammonium polysiloxane copolymers according to one of claims 1 to 5, **characterized in that** it comprises at least two repeating units V.

7. Polyammonium polysiloxane copolymers according to one of claims 1 to 5, **characterized in that** it comprises at least two repeating units V².

8. Polyammonlum polysiloxane copolymers according to one of claims 1 to 7, wherein V² is a group of the following formula
-V²*-Z²-V²*
wherein Z² is as defined above and V²* represents a divalent straight-chain, cyclic or branched, saturated, unsaturated or aromatic hydrocarbon radical having up to 40 carbon atoms, which optionally contains one or more groups selected from -O-, -NR²-, -N⁺R²₂-, wherein R² is as defined above, may contain -C(O)- and -C(S)-, and the radical V²* can optionally be substituted by one or more hydroxyl groups.

9. Polyammonlum polysiloxane copolymers according to one of claims 1 to 8, wherein the group V¹ is selected from divalent, straight-chain, cyclic or branched, saturated, unsaturated or aromatic hydrocarbon radicals having up to 600 carbon atoms, optionally containing one or more groups selected from -O-, -NR²-, -N⁺R²₂-, wherein R² is as defined above, may contain -C(O)-, -C(S)- and -Z¹-, wherein -Z¹-is a group of the following formula 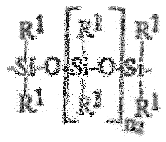 wherein
R¹ represents C₁-C₃-alkyl, fluoro(C₃-C₆)-alkyl or C₆-aryl, and
n₂ is as defined above.

10. Compound of the following formula (V): 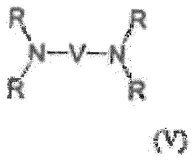 wherein R and V are as defined above, with the proviso that V contains Z¹ or Z² or an acid addition salt thereof.

11. Compound of the formula (V'): 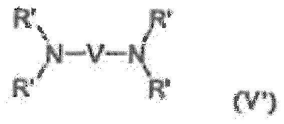 wherein each R' is an organic group containing each at least one tertiary amino group, and V is as defined above with the proviso that V contains Z¹ or Z², or an acid addition salt thereof.

12. Compound according to claim 11, wherein each R' represents a group of the following formula: 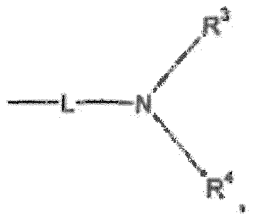 wherein R³ and R⁴ represents a methyl or an ethyl group, and L represents a C₂-C₆ alkylene group.

13. Compound according to claim 10, 11 or 12, wherein V is V².

14. A process for the preparation of the polyammonium polysiloxane copolymers according to one of claims 1 to 9, wherein the compound of formula (V) is reacted with at least one bifunctional quaternizing agent.

15. The method of claim 14, wherein the bifunctional quaternizing agents are selected from compounds having two functional groups which are selected from the group consisting of epoxy groups and bishalogenalkyl groups.

16. Use of polyammonium polysiloxane copolymers according to any one of claims 1 to 9 as well as of the compounds according to any one of claims 10 to 13 in cosmetic formulations for skin and hair care.

17. Use of polyammonium polysiloxane copolymers according to any one of claims 1 to 9 as well as of the compounds according to any one of claims 10 to 13 in polishes for the treatment and finishing of hard surfaces, In formulations for the drying of automobiles and other hard surfaces after machine washing.

18. Use of polyammonium polysiloxane copolymers according to one of the claims 1 to 9 as well as of the compounds according to any one of claims 10 to 13 for the finishing or treatment of textiles, textile fibers, paper and wood.

19. Use of polyammonium polysiloxane copolymers according to any one of claims 1 to 9 as well as of the compounds according to any one of claims 10 to 13 as an antistatic agent and/or antibacterial agent.

20. Use of polyammonium polysiloxane copolymers according to any one of claims 1 to 9 as well as of the compounds according to any one of claims 10 to 13 as separate softeners after the washing of textiles with formulations based on anionic and/or non-ionic detergents and as softener based on anionic and/or nonionic surfactants for textile washing.

21. Use of polyammonium polysiloxane copolymers according to any one of claims 1 to 9 as well as of the compounds according to any one of claims 10 to 13 as a component for improving the shine of hair in shampoos which are based on anionic, nonionic, cationic or betaine-like surfactants, In transparent or turbid "leave-on" or "rinse-off" fixatives, mousses, gel fixatives and fixative sprays.

22. Use of polyammonium polysiloxane cpoplymers according to any one of claims 1 to 9 as well as of the compounds according to any one of claims 10 to 13, as component as an aid for hair coloring or for retarding the bleeding / leaching of dyes from colored hair in shampoos which are based on anionic, non-ionic, cationic or betaine-like surfactants, in transparent or turbid "leave-on" or "rinse-off"-setting fixatives, mousses, gel fixatives and fixative sprays.

23. Use of polyammonium polysiloxane according to any one of claims 1 to 9 as well as of the compounds according to any one of claims 10 to 13 as a component for improving the hair volume in shampoos which are based on anionic, non-ionic, cationic or betaine-like surfactants, in transparent or turbid "leave-on" or "rinse-off" fixatives, mousses, gel fixatives and fixative sprays.

24. Use of polyammonium polysiloxane copolymers according to any one of claims 1 to 9 as well as of the compounds according to any one of claims 10 to 13 as a component for reducing the combing forces in shampoos which are based on anionic, non-ionic, cationic or betaine-like surfactants, In transparent or turbid "leave-on" or "rinse-off fixatives, mousses, gel fixatives and fixative sprays.

25. Use of polyammonium polysiloxane copolymers according to any one of claims 1 to 9 as well as the compounds according to any one of claims 10 to 13, as a component to improve the permanence of setting lotions In shampoos which are based on anionic, non-ionic, cationic or betaine-like surfactants, in transparent or turbid "leave-on" or "rinse-off fixatives, mousses, gel fixatives and fixative sprays.

26. Compositions containing at least one polyammonium polysiloxane copolymer according to any one of claims 1 to 9 or a compound according to any one of claims 10 to 13 together with at least one further ingredient customary for the composition.

27. The composition of claim 26 which is a detergent composition, a polishing composition or a cosmetic composition.

## Revendications

1. Copolymères de polyammonium-polysiloxane, contenant des unités répétitives de la formule (I) : 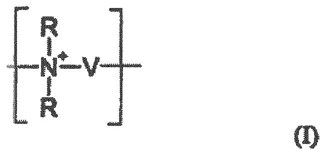 dans laquelle chacun de R représente indépendamment un groupe organique contenant chacun au moins un groupe sélectionné parmi des groupes ammonium quaternaires ou des groupes oxyde d'amine,
V est sélectionné parmi le groupe V¹ et le groupe V²,
où
V² est sélectionné parmi des résidus d'hydrocarbure bivalents ou trivalents, linéaires, cycliques ou ramifiés, saturés, insaturés ou aromatiques avec jusque 1000 atomes de carbone (les atomes de carbone du résidu de polysiloxane Z² tel que défini ci-dessous n'étant pas Inclus), qui peuvent contenir éventuellement un ou plusieurs groupes sélectionnés parmi
-O-,
-NR²-,
-N⁺R²₂-,
dans lequel R² représente un atome d'hydrogène, un résidu d'hydrocarbure monovalent, linéaire, cyclique ou ramifié, saturé, insaturé ou aromatique avec jusque 100 atomes de carbone, qui peut contenir un ou plusieurs groupes sélectionnés parmi -O-, -NH-, -C(O)- et -C(S)- et qui peut être éventuellement substitué par un ou plusieurs substituants sélectionnés parmi le groupe consistant en un groupe hydroxyle, un groupe hétérocyclique éventuellement substitué, de préférence contenant un ou plusieurs atomes d'azote, amino, alkylamino, dialkylamino, des résidus de polyéther et des résidus de polyétherester, dans lequel, lorsqu'il y a plusieurs groupes -NR²- , ils peuvent être identiques ou différents,
-C(O)-,
-C(S)- et le résidu V² peut être éventuellement substitué par un ou plusieurs groupes hydroxyle et
le résidu V² contient au moins un groupe -Z²- de la formule 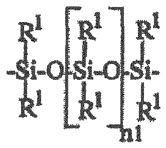 dans laquelle
R¹ peut être identique ou différent et est sélectionné parmi le groupe consistant en de l'alkyle en C₁ à C₂₂, de fluoro(C₁-C₁₀)alkyle et de l'aryle en C₆ - C₁₀ et n₁ représente 20 à 1000,
V¹ est sélectionné parmi le groupe consistant en des résidus d'hydrocarbure bivalents ou trivalents, linéaires, cycliques ou ramifiés, saturés, insaturés ou aromatiques avec jusque 1000 atomes de carbone qui peuvent contenir éventuellement un ou plusieurs groupes sélectionnés parmi
-O-,
-NR²-,
-N⁺R²₂-,
dans laquelle R² est tel que défini ci-dessus et dans laquelle les groupes R² dans les groupes V¹ et V² peuvent être identiques ou différents,
-C(O)-,
-C(S)- et
-Z¹-, dans lequel -Z¹- est un groupe de la formule 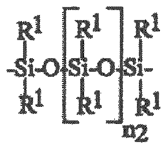 dans laquelle R¹ est tel que défini ci-dessus, dans laquelle les groupes R¹ dans les groupes V¹ et V² peuvent être identiques ou différents et
n₂ représente 0 à 19,
et le résidu V¹ peut être éventuellement substitué par un ou plusieurs groupe hydroxyle,
où les groupes V¹ et V² dans les copolymères de polyammonium-polysiloxane peuvent être identiques ou différents, à la condition qu'au moins un groupe Z¹ ou Z² est présent et
dans lesquels les charges positives résultant des groupes ammonium sont neutralisées par des anions d'acide organiques ou inorganiques.

2. Copolymères de polyammonium-polysiloxane selon la revendication 1, dans lesquels chacun de R est au moins un groupe de la formule (II) : 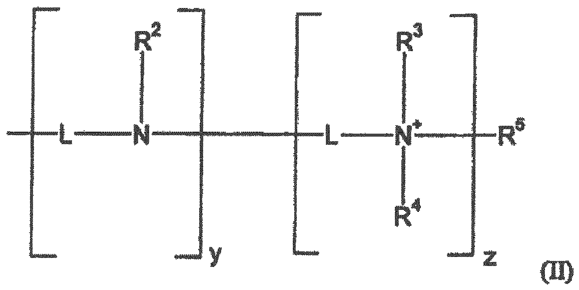 dans laquelle
chacun de L représente indépendamment un résidu d'hydrocarbure bivalent linéaire, cyclique ou ramifié, saturé, insaturé ou aromatique avec jusque 30 atomes de carbone, qui peut contenir un ou plusieurs groupes sélectionnés parmi -O-, -NH-, -C(O)- et -C(S)- et qui peut être éventuellement substitué par un ou plusieurs substituants sélectionnés parmi le groupe consistant en un groupe hydroxyle, un groupe carboxyle, un groupe carboxylate, un groupe hétérocyclique éventuellement substitué, en préférence contenant un ou plusieurs atomes d'azote, amino, alkylamino, dialkylamino, un groupe ammonium, des résidus polyéther et des résidus polyétherester,
R² est tel que défini ci-dessus,
chacun de R³, R⁴ et R⁵ est indépendamment sélectionné parmi des résidus d'hydrocarbure monovalents, linéaires, cycliques ou ramifiés, saturés, insaturés ou aromatiques avec jusque 30 atomes de carbone, qui peuvent contenir un ou plusieurs groupes sélectionné parmi -O-, -NH-, -C(O)- et -C(S)- et qui peuvent être éventuellement substitués par un ou plusieurs substituants sélectionnés parmi le groupe consistant un groupe hydroxyle, un groupe carboxyle, un groupe carboxylate, un groupe hétérocyclique éventuellement substitué, en préférence contenant un ou plusieurs atomes d'azote, amino, alkylamino, dlalkylamino, un groupe ammonium, des résidus polyéther et des résidus polyétherester et
R³ ou R⁴ peut être de l'oxygène pour former un oxyde d'amine et
z peut être de 1 à 10 et y peut être de 0 à 10.

3. Copolymères de polyammonium-polysiloxane selon l'une des revendications 1 ou 2, dans lesquels chacun de R est un groupe de la formule (III) 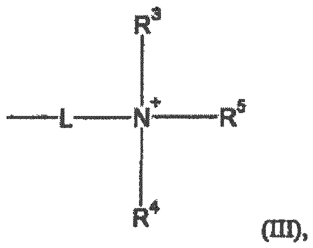 dans laquelle L, R³, R⁴ et R⁵ sont tels que définis ci-dessus.

4. Copolymères de polyammonium-polysiloxane selon l'une des revendications 1 à 3, dans lesquels chacun de R est au moins un groupe oxyde d'amine de la formule (IV) : 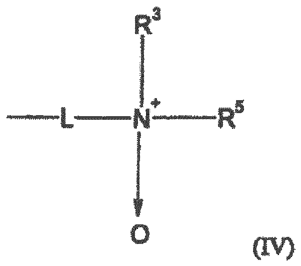 dans laquelle L, R³ et R⁵ sont tels que définis ci-dessus.

5. Copolymères de polyammonium-polysiloxane selon l'une des revendications 2 à 4, dans lesquels L est sélectionné parmi des groupes alkylène en C₁ à C₁₀, qui peuvent être linéaires, ramifiés ou cycliques,

6. Copolymères de polyammonium-polysiloxane selon l'une des revendications 1 à 5, **caractérisés en ce qu'**ils ont au moins deux unités répétitives V.

7. Copolymères de polyammonium-polysiloxane selon l'une des revendications 1 à 5, **caractérisés en ce qu'**ils ont au moins deux unités répétitives V².

8. Copolymères de polyammonium-polysiloxane selon l'une des revendications 1 à 7, dans lesquels V² est un groupe de la formule
**-V²*-Z²-V²*-**
dans laquelle Z² est tel que défini ci-dessus et V^{2*} représente un résidu d'hydrocarbure bivalent linéaire, cyclique ou ramifié, saturé, insaturé ou aromatique avec jusque 40 atomes de carbone, qui peut contenir éventuellement un ou plusieurs groupes sélectionnés parmi -O-, -NR²-, - N⁺R²₂-, dans laquelle R² est tel que défini ci-dessus, -C(O)- et -C(S)- et le résidu V^{2*} peut être éventuellement substitué par un ou plusieurs groupes hydroxyle.

9. Copolymères de polyammonium-polysiloxane selon l'une des revendications 1 à 8, dans lesquels le groupe V¹ est sélectionné parmi des résidu d'hydrocarbure bivalents, linéaires, cycliques ou ramifiés, saturés, insaturés ou aromatiques avec jusque 600 atomes de carbone, qui peuvent éventuellement contenir un ou plusieurs groupes sélectionnés parmi -O-, -NR²-, -N⁺R²₂-, dans laquelle R² est tel que défini ci-dessus, -C(O)-, -C(S)- et -Z¹-, dans laquelle -Z¹- est un groupe de la formule 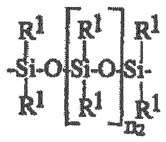 dans laquelle R¹ est un alkyle en C₁ à C₃, un fluoro(C₃-C₆)alkyle ou un aryle en C₆ et n₂ est tel que défini ci-dessus.

10. Composé de la formule (V) : 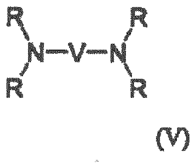 dans laquelle R et V sont tels que définis ci-dessus, à la condition que V contient Z¹ ou Z², ou un sel de l'addition d'un acide de celui-ci,

11. Composé de la formule (V') : 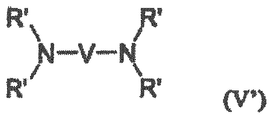 dans laquelle chacun de R' est un groupe organique contenant chacun au moins un groupe amino tertiaire et V est tel que défini ci-dessus, à la condition que V contient Z¹ ou Z², ou un sel de l'addition d'un acide de celui-ci.

12. Composé selon la revendication 11, dans lequel chacun de R' représente un groupe de la formule suivante : 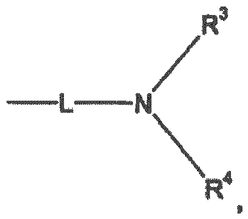 dans laquelle R³ et R⁴ représentent un groupe méthyle ou éthyle et L représente un groupe alkylène en C2 à C6.

13. Composé selon l'une des revendications 10, 11 ou 12, dans lequel V est V^{2.}

14. Procédé de production des copolymères de polyammonlum-polysiloxane selon l'une des revendications 1 à 9, dans lequel le composé de la formule (V) est fait réagir avec au moins un moyen de quarternisation bifonctionnel.

15. Procédé selon la revendication 14, dans lequel les moyens de quarternisation bifonctionnels sont sélectionnés parmi des composés ayant deux groupes fonctionnels qui sont sélectionnés parmi le groupe consistant en des groupes époxy et des groupes bishalogènealkyle.

16. Utilisation des copolymères de polyammonium-polysiloxane selon l'une des revendications 1 à 9 ainsi que des composés selon l'une des revendications 10 à 13 dans des formulations cométiques pour les soins de la peau et des cheveux.

17. Utilisation des copolymères de polyammonium-polysiloxane selon l'une des revendications 1 à 9 ainsi que des composés selon l'une des revendications 10 à 13 dans des pâtes à polir pour le traitement et l'équipement des surfaces dures, dans des formulations pour le séchage des voitures automobiles et d'autres surfaces dures après le levage mécanique.

18. Utilisation des copolymères de polyammonium-polysiloxane selon l'une des revendications 1 à 9 ainsi que des composés selon l'une des revendications 10 à 13 pour l'équipement et le traitement des textiles, des fibres textiles, du papier et du bois.

19. Utilisation des copolymères de polyammonium-polysiloxane selon l'une des revendications 1 à 9 ainsi que des composés selon l'une des revendications 10 à 13 en tant qu'agent antistatique et /ou agent antibactérien.

20. Utilisation des copolymères de polyammonium-polysiloxane selon l'une des revendications 1 à 9 ainsi que des composés selon l'une des revendications 10 à 13 en tant qu'assouplissants séparés après le lavage des textiles avec des formulations à base des détergents anioniques et/ou non-ionique et en tant qu'assouplissant dans des formulations à base des tensioactifs anioniques et/ou non-ioniques pour le lavage des textiles,

21. Utilisation des copolymères de polyammonium-polysiloxane selon l'une des revendications 1 à 9 ainsi que des composés selon l'une des revendications 10 à 13 en tant que composant pour l'amélioration du lustre des cheveux dans des shampoings, qui sont à base des tensioactifs anioniques, non-ioniques, cationiques ou de structure bétaïne, dans des agents raffermissants, des mousse coiffantes, des gels coiffants ou des sprays coiffants « leave-on » ou « rinse-off » transparents ou turbides.

22. Utilisation des copolymères de polyammonium-polysiloxane selon l'une des revendications 1 à 9 ainsi que des composés selon l'une des revendications 10 à 13 en tant que composant en tant qu'agent auxiliaire pour la coloration des cheveux ou pour le retard de la lixiviation/perte par rinçage des colorants des cheveux colorés dans des shampooings, qui sont à base des tensioactifs anioniques, non-ioniques, cationiques ou de structure bétaïne, dans des agents raffermissants, des mousse coiffantes, des gels coiffants ou des sprays coiffants « leave-on » ou « rinse-off » transparents ou turbides.

23. Utilisation des copolymères de polyammonium-polysiloxane selon l'une des revendications 1 à 9 ainsi que des composés selon l'une des revendications 10 à 13 en tant que composant pour l'amélioration du volume des cheveux dans des shampooings, qui sont à base des tensioactifs anioniques, non-ioniques, cationiques ou de structure bétaïne, dans des agents raffermissants, des mousse coiffantes, des gels coiffants ou des sprays coiffants « leave-on » ou « rinse-off » transparents ou turbides.

24. Utilisation des copolymères de polyammonium-polysiloxane selon l'une des revendications 1 à 9 ainsi que des composés selon l'une des revendications 10 à 13 en tant que composant pour la réduction des pouvoirs lissants dans des shampooings, qui sont à base des tensioactifs anioniques, non-ioniques, cationiques ou de structure bétaïne, dans des agents raffermlssants, des mousse coiffantes, des gels coiffants ou des sprays coiffants « leave-on » ou « rinse-off » transparents ou turbides.

25. Utilisation des copolymères de polyammonium-polysiloxane selon l'une des revendications 1 à 9 ainsi que des composés selon l'une des revendications 10 à 13 en tant que composant pour l'amélioration de la permanence des agents raffermissants dans des shampooings, qui sont à base des tensioactifs anioniques, non-ioniques, cationiques ou de structure bétaïne, dans des agents raffermlssants, des mousse coiffantes, des gels coiffants ou des sprays coiffants « leave-on » ou « rinse-off » transparents ou turbides.

26. Composition contenant au moins un copolymère de polyammonium-polysiloxane selon l'une des revendications 1 à 9 ou un composé selon l'une des revendications 10 à 13 avec au moins un ingrédient habituel pour la compositions.

27. Composition selon la revendication 26, qui est une composition détergente, une composition des pâtes à polir ou une composition cosmétique.
